(19) Europäisches Patentamt
European Patent Office
Office européen des brevets

(11) Veröffentlichungsnummer: **0 112 284**
**A2**

(12) # EUROPÄISCHE PATENTANMELDUNG

(21) Anmeldenummer: **83810521.1**

(22) Anmeldetag: **14.11.83**

(51) Int. Cl.³: **C 07 D 405/06**
C 07 D 233/60, A 61 K 31/415
C 07 D 319/06, C 07 D 317/22
C 07 D 317/16

(30) Priorität: **16.11.82 US 442062**

(43) Veröffentlichungstag der Anmeldung:
**27.06.84 Patentblatt 84/26**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Hubele, Adolf, Dr.**
**Obere Egg 9**
**CH-4312 Magden(CH)**

(72) Erfinder: **Riebli, Peter**
**Bünten 17**
**CH-4446 Buckten(CH)**

(54) **Neue Arylphenylether-derivate.**

(57) Arylphenylether-derivate der Formel I

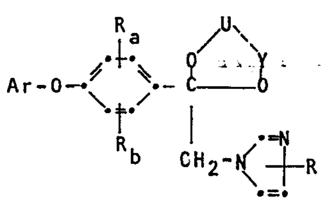

(I),

worin

R für $C_1$-$C_6$-Alkyl steht;

$R_a$ und $R_b$ unabhängig voneinander Wasserstoff, Halogen oder $C_1$-$C_3$-Alkyl bedeuten;

Ar unsubstituiertes oder durch Halogen, $C_1$-$C_3$-Alkyl, $C_1$-$C_3$-Alkoxy und/oder Trifluormethyl ein- oder mehrfach substituiertes Phenyl bedeutet;

U und V unabhängig voneinander für gegebenenfalls durch Halogen oder $C_1$-$C_6$-Alkoxy substituiertes $C_1$-$C_{12}$-Alkyl stehen oder zusammen eine der folgenden Alkylenbrücken

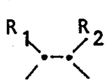 , 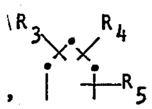 oder 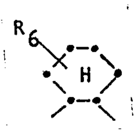

bilden, wobei

$R_1$ und $R_2$ unabhängig voneinander für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_3$-Alkyl substituiertes Phenyl oder für die Gruppe $-CH_2-Z-R_7$ stehen, wobei

Z Sauerstoff oder Schwefel bedeutet und

$R_7$ für Wasserstoff, $C_1$-$C_8$-Alkyl, ein durch $C_1$-$C_2$-Alkoxy substituiertes $C_1$-$C_8$-Alkyl, $C_3$-$C_4$-Alkenyl, 2-Propinyl, 3-Halogen-2-propinyl, Phenyl, ein durch Halogen, $C_1$-$C_3$-Alkyl, $C_1$-$C_3$-Alkoxy, Nitro und/oder $CF_3$ ein- oder mehrfach substituiertes Phenyl, Benzyl oder ein durch Halogen, $C_1$-$C_3$-Alkyl und/oder $C_1$-$C_3$-Alkoxy ein- oder mehrfach substituiertes Benzyl steht;

$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$-$C_3$-Alkyl steht;

unter Einschluss ihrer Säureadditionssalze haben anxiolytische, antikonvulsive und/oder antimykotische Eigenschaften und können als Arzneimittelwirkstoffe verwendet werden. Die Arylphenylether-derivate der Formel I werden hergestellt, indem man z.B. eine Verbindung der Formel II

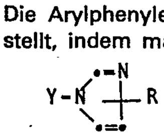

(II),

./...

EP 0 112 284 A2

worin Y Wasserstoff oder ein Metallkation darstellt, mit einer Verbindung der Formel III

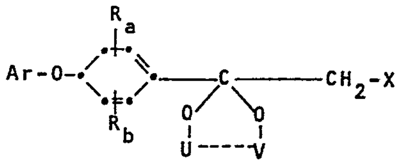

(III),

worin X eine nukleofuge Abgangsgruppe bedeutet, kondensiert.

CIBA-GEIGY AG
Basel (Schweiz)

4-14621/13393

## Neue Arylphenylether-derivate

Die vorliegende Erfindung betrifft substituierte Arylphenylether-derivate der nachstehenden Formel I sowie deren Säureadditionssalze. Sie betrifft ferner die Herstellung dieser Verbindungen sowie pharmazeutische Präparate, die als Wirkstoff mindestens eine der Verbindungen der Formel I enthalten, die Herstellung dieser Präparate und ihre Verwendung sowie ein Verfahren zur Behandlung von Epilepsien sowie von Angst- und Erregungszuständen.

Die erfindungsgemässen Arylphenylether-derivate haben die Formel I,

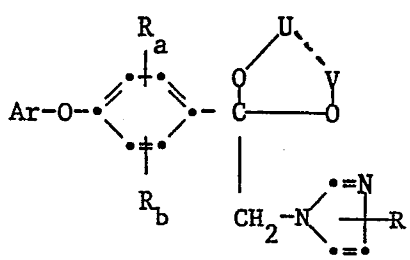

(I) ,

worin

R für $C_1$-$C_6$-Alkyl steht;

$R_a$ und $R_b$ unabhängig voneinander Wasserstoff, Halogen oder $C_1$-$C_3$-Alkyl bedeuten;

Ar unsubstituiertes oder durch Halogen, $C_1$-$C_3$-Alkyl, $C_1$-$C_3$-Alkoxy und/oder Trifluormethyl ein- oder mehrfach substituiertes Phenyl bedeutet;

U und V unabhängig voneinander für gegebenenfalls durch Halogen oder $C_1$-$C_6$-Alkoxy substituiertes $C_1$-$C_{12}$-Alkyl stehen oder zusammen eine der folgenden Alkylenbrücken

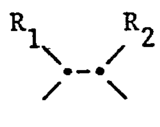 , 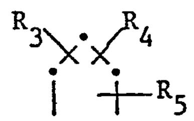 oder 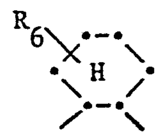 bilden, wobei

$R_1$ und $R_2$ unabhängig voneinander für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_3$-Alkyl substituiertes Phenyl oder für die Gruppe $-CH_2-Z-R_7$ stehen, wobei

Z Sauerstoff oder Schwefel bedeutet und

$R_7$ für Wasserstoff, $C_1$-$C_8$-Alkyl, ein durch $C_1$-$C_2$-Alkoxy substituiertes $C_1$-$C_8$-Alkyl, $C_3$-$C_4$-Alkenyl, 2-Propinyl, 3-Halogen-2-propinyl, Phenyl, ein durch Halogen, $C_1$-$C_3$-Alkyl, $C_1$-$C_3$-Alkoxy, Nitro und/oder $CF_3$ ein- oder mehrfach substituiertes Phenyl, Benzyl oder ein durch Halogen, $C_1$-$C_3$-Alkyl und/oder $C_1$-$C_3$-Alkoxy ein- oder mehrfach substituiertes Benzyl steht;

$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$-$C_3$-Alkyl steht;

unter Einschluss ihrer Säureadditionssalze.

Der Substituent Ar hat beispielsweise die Formel

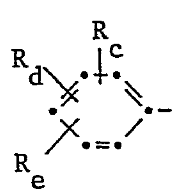

worin $R_c$, $R_d$ und $R_e$ unabhängig voneinander für Wasserstoff, Halogen, $C_1$-$C_3$-Alkyl, $C_1$-$C_3$-Alkoxy oder Trifluormethyl stehen.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl sowie ihre Isomeren wie z.B. Isopropyl, Isobutyl, tert.-Butyl, sek.-Butyl, Isopentyl usw. Alkenyl steht z.B. für Propenyl-(1), Allyl, Butenyl-(1), Butenyl-(2) oder Butenyl-(3). Halogen soll hier und im fogenden Fluor, Chlor, Brom oder Jod, vorzugsweise Chlor oder Brom, bedeuten.

Die Erfindung betrifft sowohl die freien Verbindungen der Formel I, als auch deren Additionssalze mit anorganischen und organischen Säuren.

Erfindungsgemässe Salze sind insbesondere Additionssalze mit physiologisch unbedenklichen anorganischen oder organischen Säuren.

Physiologisch unbedenkliche Säuren, d.h. pharmazeutisch verwendbare Säuren, sind bespielsweise pharmazeutisch verwendbare Mineralsäuren, wie Halogenwasserstoffsäuren, z.B. Chlor-, Brom- oder Jodwasserstoffsäure, Salpetersäure, Schwefelsäure oder Phosphorsäure, pharmazeutisch verwendbare Carbon- und Sulfonsäuren, wie aliphatische Mono- und gegebenenfalls hydroxylierte Dicarbonsäuren, z.B. Essigsäure, Fumarsäure, Maleinsäure, Apfelsäure oder Weinsäure, ebenso aliphatische oder aromatische Sulfonsäuren, wie Niederalkan- oder gegebenenfalls substituierte Benzolsulfonsäure, z.B. Methan-, Ethan-, Benzol-, p-Toluol- und p-Brombenzolsulfonsäure, ferner Sulfaminsäuren, z.B. N-Cyclohexylsulfaminsäure.

Die Verbindungen der Formel I sind bei Raumtemperatur stabile Oele, Harze oder Feststoffe, die sich durch wertvolle pharmakologische,

insbesondere antikonvulsive und/oder anxiolotische, ebenso antidepressive und/oder antimykotische Eigenschaften auszeichnen. Sie lassen sich daher als Antikonvulsiva und/oder Anxiolytika, ebenso als Antidepressiva und/oder Antimykotika, beispielsweise zur Bekämpfung von an Warmblütern parasitären Pilzen und/oder zur Behandlung von verschiedenen Formen der Epilepsie, von Angst-, Spannungs- und Erregungszuständen und/oder von manischen Gemütszuständen einsetzen.

Die Erfindung betrifft beispielsweise Verbindungen der Formel I, worin R, $R_a$ und $R_b$ die angegebenen Bedeutungen haben, Ar eine Gruppe der Formel

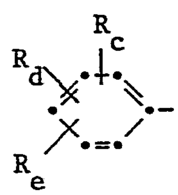

darstellt, in der $R_c$, $R_d$ und $R_e$ unabhängig voneinander Wasserstoff, Halogen, Trifluormethyl, $C_1$-$C_3$-Alkyl oder $C_1$-$C_3$-Alkoxy bedeuten, und U und V unabhängig voneinander $C_1$-$C_6$-Alkyl oder durch Halogen bzw. $C_1$-$C_2$-Alkoxy substituiertes $C_2$-$C_4$-Alkyl oder gemeinsam eine der folgenden Alkylengruppen

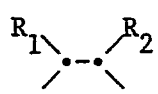 oder 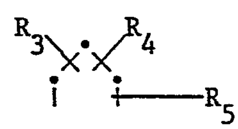

darstellen, worin $R_1$, $R_2$, $R_3$, $R_4$ und $R_5$ unabhängig voneinander Wasserstoff oder $C_1$-$C_4$-Alkyl, wobei die Gesamtzahl der Kohlenstoffatome von $R_3$, $R_4$ und $R_5$ 6 nicht übersteigt, oder $R_1$ Wasserstoff und $R_2$ eine Gruppe der Formel $CH_2OR_7$ darstellt, in der $R_7$ $C_1$-$C_4$-Alkyl, durch $C_1$-$C_2$-Alkoxy substituiertes $C_2$-$C_4$-Alkyl, $C_3$-$C_4$-Alkenyl oder Prop-2-inyl bedeutet, unter Einschluss ihrer Säureadditionssalze.

Verbindungen mit ausgesprochen nützlichen antikonvulsiven und anxiolytischen Eigenschaften sind solche, worin R die unter der Formel I angegebene Bedeutung hat, $R_a$ und $R_b$ unabhängig voneinander Wasserstoff, Methyl, Chlor oder Brom bedeuten, Ar unsubstituiertes oder durch Halogen, Methyl oder Trifluormethyl substituiertes Phenyl bedeutet und U und V unabhängig voneinander $C_1$-$C_3$-Alkyl, gegebenenfalls durch Halogen oder $C_1$-$C_2$-Alkoxy substituiertes $C_2$-$C_3$-Alkyl oder zusammen eine der folgenden Alkylengruppen

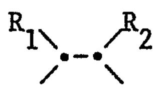

oder

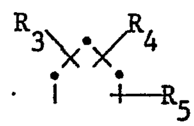

bilden, worin $R_1$, $R_2$, $R_3$, $R_4$ und $R_5$ unabhängig voneinander für Wasserstoff oder $C_1$-$C_4$-Alkyl stehen, wobei die Gesamtzahl der Kohlenstoffatome in $R_3$, $R_4$ und $R_5$ die Zahl 4 nicht übersteigen sollen.

Eine weitere bevorzugte Gruppe von ausgesprochen antikonvulsiven und anxiolytischen wirkenden Verbindungen sind die hervorzuheben, worin R die unter der Formel I angegebene Bedeutung hat, $R_a$ und $R_b$ Wasserstoff bedeuten, Ar für unsubstituiertes oder durch Halogen oder Methyl substituiertes Phenyl und U sowie V gemeinsam eine Gruppe der Formel

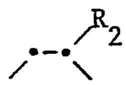 oder 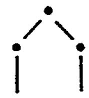

darstellen, in der $R_2$ $C_1$-$C_4$-Alkyl, wie Methyl oder Ethyl, Hydroxy-$C_1$-$C_3$-alkyl, wie Hydroxymethyl oder 2-Hydroxyethyl, oder $C_1$-$C_2$-Alkoxy-$C_1$-$C_2$-alkyl, wie Methoxymethyl oder Ethoxymethyl, bedeuten.

Folgende Verbindungen seien als Beispiele für bezüglich ihrer antikonvulsiven, antidepressiven und/oder anxiolytischen Eigenschaften besonders bevorzugte Verbindungen genannt:

2-[p-(p-Chlorphenoxy)phenyl]-2-[1-(2-methylimidazolyl)methyl]-4-ethyl-1,3-dioxolan,

2-(p-Phenoxyphenyl)-2-[1-(2-methylimidazolyl)methyl]-4-methyl-1,3-dioxan,

2-[4-(p-Chlorphenoxy)-2-methyl-phenyl]-2-[1-(2-methylimidazolyl)-methyl]-4-methyl-1,3-dioxolan,

2-[4-(p-Chlorphenoxy)-2-methyl-phenyl]-2-[1-(2-methylimidazolyl)-methyl]-4-ethyl-1,3-dioxolan,

2-[p-(m-Chlorphenoxy)phenyl]-2-[1-(2-methylimidazolyl)methyl]-4-ethyl-1,3-dioxolan,

2-(2-Chlor-4-phenoxy-phenyl)-2-[1-(2-methylimidazolyl)methyl]-4-ethyl-dioxolan,

2-[p-(p-Chlorphenoxy)phenyl]-2-[1-(2-methylimidazolyl)methyl]-1,3-dioxan,

2-[p-(4-Chlor-3-methyl-phenoxy)phenyl]-2-[1-(2-methylimidazolyl)-methyl]-4-methoxymethyl-1,3-dioxolan,

2-(p-Phenoxyphenyl)-2-[1-(2-methylimidazolyl)methyl]-4-propyl-dioxolan und

2-[p-(2,4-Dichlorphenoxy)phenyl]-2-[1-(2-methylimidazolyl)-methyl]-4-ethyl-1,3-dioxolan

und jeweils ihre pharmazeutisch verwendbaren Säureadditionssalze.
Die Erfindung betrifft ganz speziell diese, Verfahren zu ihrer Herstellung, dieselben enthaltende pharmazeutische Präparate und ihre Verwendung als Arzneimittelwirkstoffe.

Die Verbindungen der Formel I können hergestellt werden, indem man

A) eine Verbindung der Formel II

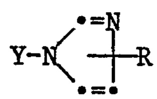

(II) ,

worin Y Wasserstoff oder ein Metallkation darstellt, mit einer Verbindung der Formel III

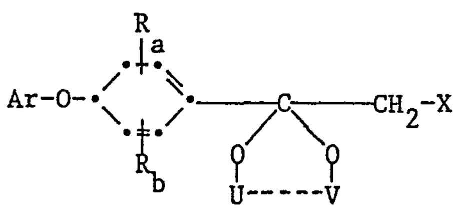

(III),

worin X eine nukleofuge Abgangsgruppe bedeutet, kondensiert oder

B) in einer Verbindung der Formel IV

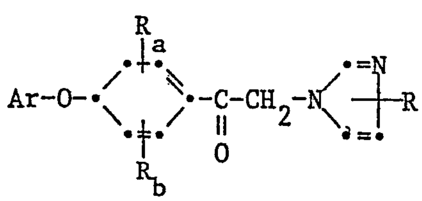

(IV)

die Carbonylgruppe in eine Gruppe der Formel V

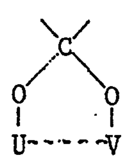

(V)

überführt, oder

C) zur Herstellung von Verbindungen der Formel I, worin U und V gemeinsam eine Gruppe der Formel $-CH_2-CH(CH_2ZR_7')-$ darstellen und $R_7'$ einen von Wasserstoff verschiedenen Rest $R_7$ bedeutet, Verbindungen der

Formeln VI und VII

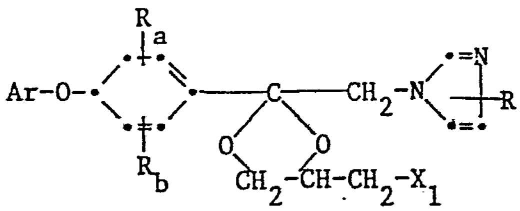 (VI) und $X_2 - R_7$ (VII),

worin einer der Reste $X_1$ und $X_2$ gegebenenfalls in Salzform vorliegendes Hydroxy oder Mercapto, z.B. der Formel -Z-Y, in der Y Wasserstoff oder vorzugsweise ein Metallkation ist, und der andere eine nukleofuge Abgangsgruppe X bedeutet oder sowohl $X_1$ als auch $X_2$ Hydroxygruppen darstellen, miteinander kondensiert oder

D) Verbindungen der Formeln VIII und IX

$Ar-X_3$ (VIII) und 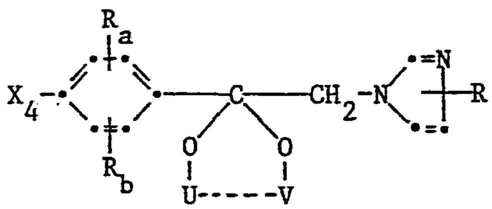 (IX),

worin einer der Reste $X_3$ und $X_4$ eine Gruppe -O-Y, in der Y Wasserstoff oder vorzugsweise ein Metallkation ist, und der andere einen gegen Aryloxy austauschbaren Rest bedeutet, miteinander kondensiert oder

E) eine Verbindung der Formel

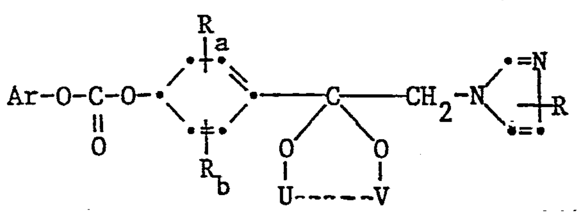 (X)

intramolekular decarboxyliert und, wenn erwünscht, eine verfahrensgemäss erhältliche Verbindung in eine andere Verbindung der Formel I umwandelt und/oder eine verfahrensgemäss erhältliche freie Verbindung in ein Säureadditionssalz, ein verfahrensgemäss erhältliches Säureadditionssalz in die freie Verbindung oder in ein anderes Säureadditionssalz überführt.

Metallkationen Y sind dabei beispielsweise Alkalimetall-, z.B. Lithium-, Natrium- oder Kaliumkationen, oder Erdalkalimetall-, z.B. Magnesium-, Calcium-, Strontium- oder Bariumkationen.

Nukleofuge Abgangsgruppen sind beispielsweise reaktionsfähige veresterte Hydroxygruppen, wie mit einer Halogenwasserstoffsäure, z.B. mit Fluor, Chlor-, Brom- oder Jodwasserstoffsäure, oder einer Niederalkan-, gegebenenfalls substituierten Benzol- oder Halogensulfonsäure, z.B. mit Methan-, Ethan-, Benzol-, p-Toluol- oder Fluorsulfonsäure, veresterte Hydroxygruppen.

Die Umsetzung eines Azols der Formel II, worin R die unter Formel I angegebene Bedeutung hat und Y bevorzugt ein Metallatom, insbesondere ein Alkalimetallatom darstellt, mit einer Verbindung der Formel III, worin Ar, $R_a$, $R_b$, U und V die unter Formel I angegebenen Bedeutungen haben und X beispielsweise für Halogen, insbesondere Chlor, Brom oder Jod, oder Benzolsulfonyloxy, p-Tosyloxy, Trifluoracetyloxy oder bevorzugt Niederalkylsulfonyloxy wie z.B. Mesyloxy, bedeutet, wird bevorzugt in einem relativ polaren, jedoch reaktionsinerten organischen Lösungsmittel durchgeführt, z.B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, wie aliphatischen oder aromatischen Kohlenwasserstoffen, z.B. Benzol, Toluol, Xylol, Hexan, Petrolether, Chlorbenzol, Nitrobenzol u.a. verwendet werden.

Bedeutet X Chlor oder Brom, so kann man zweckmässig Alkalijodid (wie NaJ oder KJ) zur Beschleunigung der Reaktion zusetzen. Erhöhte Temperaturen von 0 bis 220°C, bevorzugt 80-170°C, sind vorteilhaft. Zweckmässig wird das Reaktionsgemisch unter Rückfluss erhitzt.

In den Fällen, in denen in Formel II Y für Wasserstoff steht, wird das Verfahren in Gegenwart einer Base durchgeführt. Beispiele solcher Basen sind anorganische Basen wie die Oxide, Hydroxide, Hydride, Carbonate und Hydrogencarbonate von Alkali und Erdalkalimetallen sowie z.B. tert. Amine wie Triethylamin, Triethylendiamin, Piperidin, Pyridin, 4-Dimethylaminopyridin, 4-Pyrrolidylpyridin usw.

Bei dieser und den folgenden Herstellungsvarianten können die Zwischen- und Endprodukte aus dem Reaktionsmedium isoliert und, falls erwünscht, auf eine der allgemein üblichen Methoden gereinigt werden, z.B. durch Extraktion, Kristallisation, Chromatographie, Destillation usw.

Die Ueberführung der Carbonylgruppe in Verbindungen der Formel IV in die Gruppe der Formel V erfolgt durch Umsetzung mit einem Orthocarbonsäure-tri-$C_1$-$C_{12}$-alkylester, dessen $C_1$-$C_{12}$-Alkylgruppen gegebenenfalls durch Halogen oder $C_1$-$C_6$-Alkoxy substituiert sind, oder in Gegenwart einer Säure mit mindestens 2 Mol eines einwertigen Alkohols der Formel U-OH (Va), wobei Verbindungen der Formel I erhalten werden, worin U und V gleiche, gegebenenfalls substituierte $C_1$-$C_{12}$-Alkylgruppen bedeuten, oder durch Umsetzung mit einem Diol der Formel Vb

HO-U-----V-OH (Vb),

wobei Verbindungen der Formel I erhalten werden, worin U und V gemeinsam eine der eingangs definierten Alkylenbrücken darstellen. Dabei haben Ar, R, $R_a$, $R_b$, U und V die unter Formel I angegebenen Bedeutungen.

Diese Ketalisierungsreaktion kann analog zu bereits bekannten Ketalisierungs-Reaktionen durchgeführt werden, beispielsweise analog zur Herstellung von 2-Brommethyl-2,4-diphenyl-1,3-dioxolan [Synthesis, 1974 (I), 23].

Bei der bevorzugten Ausführung der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z.B. Benzol, Toluol, Xylol, Chloroform oder Tetrachlorkohlenstoff in Frage, wobei zur Beschleunigung der Reaktion ein Zusatz einer starken Säure, wie z.B. p-Toluolsulfonsäure, vorteilhaft sein kann. Verwendbare organische Lösungsmittel sind in diesem Fall z.B. aromatische Kohlenwasserstoffe, wie Benzol, Toluol, Xylol usw., gesättigte Kohlenwasserstoffe, wie n-Hexan oder gesättigte halogenierte Kohlenwasserstoffe wie z.B. 1,1,1-Trichlorethan.

Es sind auch weitere Wege der Ketalisierung durchführbar, z.B. indem man ein Keton IV, das mit einem von Alkanolen bzw. Diolen der Formeln Va bzw. Vb verschiedenen Alkohol oder Phenol ketalisiert ist, umsetzt und dieses durch Reaktion mit überschüssigem Alkanol Va bzw. dem Diol Vb zu (I) umketalisiert. Das Ausgangsprodukt ist z.B. gemäss einer der Verfahrensvarianten A), D) und E) zugänglich.

Die Herstellung von Verbindungen der Formel I, worin Substituenten U und V gemäss Variante C) zusammen für $-CH_2-CH(CH_2ZR_7)-$ stehen, erfolgt beispielsweise durch Reaktion einer Verbindung der Formel VI mit einer Verbindung der Formel VII, worin $X_1$ eine Gruppe -ZH und $X_2$ eine Gruppe X bedeutet. Die Reaktion wird bevorzugt in reaktions-inerten organischen Lösungsmitteln durchgeführt. Es eignen sich hierzu z.B. N,N-Dimethylformamid, N,N-Dimethylacetamid, Hexamethylphosphortriamid, Dimethylsulfoxid, 4-Methyl-3-pentanon usw. Auch Gemische mit anderen reaktionsinerten Lösungsmitteln, z.B. mit aromatischen Kohlenwasserstoffen wie Benzol, Toluol, Xylol usw. können verwendet werden. In manchen Fällen kann es sich als vorteilhaft erweisen, zur Beschleunigung der Reaktionsgeschwindigkeit in Gegenwart einer Base zu arbeiten. Solche Basen sind z.B. Alkalimetallhydride oder Alkalimetallcarbonate. In gewissen Fällen kann es auch von Vorteil sein, dass man die Verbindung VI zuerst auf bekannte Art in ein geeignetes Metall-Salz überführt.

Dies geschieht vorzugsweise durch Reaktion von VI mit einer Na-Verbindung, z.B. Natriumhydrid, Natriumhydroxid usw. Anschliessend wird dieses Salz von VI mit der Verbindung der Formel VII umgesetzt. Zur Erhöhung der Reaktionsgeschwindigkeit kann in manchen Fällen auch bei erhöhter Temperatur, vorzugsweise 80°C bis 130°C, bzw. am Siedepunkt des Lösungsmittels gearbeitet werden.

In analoger Weise kann man auch Verbindungen der Formeln VI und VII, worin $X_1$ eine Gruppe X und $X_2$ eine Gruppe -ZH ist, umsetzen.

Bei der zu Produkten der Formel I, worin Z Sauerstoff ist, führenden Kondensations-Reaktion von Verbindungen der Formeln VI und VII, worin $X_1$ und $X_2$ Hydroxy darstellen, können die Reaktanden in einem geeigneten Lösungsmittel unter Rückfluss erhitzt werden, wobei gleichzeitig das entstehende Wasser azeotrop aus dem Reaktionsgemisch abdestilliert wird. Als Lösungsmittel kommen aromatische Kohlenwasserstoffe, wie Toluol oder Alkohol HO-$R_7$ selbst in Frage. Bei dieser Reaktion arbeitet man zweckmässigerweise in Gegenwart einer starken Säure, z.B. p-Toluolsulfonsäure.

Bei der Variante D) geht man vorzugsweise von Verbindungen der Formeln VIII und IX aus, worin $X_3$ eine Gruppe -OY und $X_4$ eine nukleofuge Abgangsgruppe ist oder umgekehrt, $X_3$ die nukleofuge Abgangsgruppe darstellt und $X_4$ die Gruppe -OY bildet. Hierbei haben $R_a$, $R_b$, U, V, R und Ar die unter Formel I angegebenen Bedeutungen; Y steht bevorzugt für Wasserstoff. Die Reaktion wird vorteilhafterweise unter den bei Variante A) beschriebenen Bedingungen durchgeführt.

Bei der Variante E) wird die zu decarboxylierende Verbindung der Formel X, zugänglich durch Ketalisierung einer Verbindung der Formel XI, analog wie unter B) beschrieben,

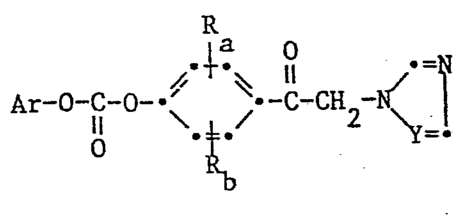

(XI),

die ihrerseits durch Umsetzung einer Verbindung der Formel Ar-OH XII mit einem difunktionellen Derivat der Kohlensäure, z.B. mit Phosgen, einem Halogenameisensäureniederalkylester oder einem Diniederallyl- oder Diphenylcarbonat und Weiterreaktion mit einer Verbindung der Formel XIII

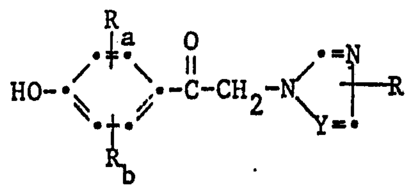

(XIII)

erhalten werden kann, trocken oder in einem hochsiedenden Lösungsmittel, wie einem hochsiedenden Ether, z.B. Diphenylether oder Ethylenglykoldimethylether, auf etwa 120° bis 220°C erhitzt.

Verfahrensgemäss erhältliche Verbindungen können nach an sich bekannten Methoden in andere Verbindungen der Formel I überführt werden.

So kann man beispielsweise verfahrensgemäss erhältliche Verbindungen zu anderen Verbindungen der Formel I umketalisieren. Beispielsweise kann man in Verbindungen der Formel I, worin U und V gleiche, gegebenenfalls substituierte, $C_1$-$C_{12}$-Alkylreste U darstellen, durch Umsetzung mit 1 Mol eines anderen, gegebenenfalls substituierten $C_1$-$C_{12}$-Alkanols der Formel V-OH (Vc), eine Gruppe U durch eine Gruppe V oder durch Umsetzung mit einem Diol der Formel Vb beide Gruppen U durch einen zweiwertigen Rest ersetzen. Die Umketalisierung erfolgt in üblicher Weise, beispielsweise in Gegenwart eines sauren Kondensationsmittels, wie einer Mineral-, Sulfon- oder starken Carbonsäure, z.B. von Chlor- oder Bromwasserstoffsäure, Schwefelsäure, p-Toluolsulfonsäure oder Trifluoressigsäure, vorteilhaft unter destillativer bzw. azeotropdestillativer Entfernung leichtflüchtiger Reaktionsprodukte.

Ferner kann man in die carbocyclischen Arylteile verfahrensgemäss erhältlicher Verbindungen gegebenenfalls zusätzliche Substituenten

in den Rest Ar und/oder die Gruppen $R_a$ bzw. $R_b$ einführen. So kann man z.B. durch Umsetzung mit einem Halogen in Gegenwart einer Lewissäure, wie einem Eisen-, Zink-, Bor- oder Antimonhalogenides, oder durch Behandlung mit N-Chlorsuccinimid, Halogen einführen.

Ferner kann man Nitrogruppen, z.B. mittels geeigneter komplexer Hydride, z.B. mit Lithiumaluminiumhydrid, zu Aminen reduzieren, diese, z.B. mittels salpetriger Säure, diazotieren und die gebildete Diazoniumgruppe in üblicher Weise durch Halogen oder Alkoxy ersetzen. Ebenso kann man Halogen durch Umsetzung mit einer Alkylmetallverbindung, z.B. mit einem Alkyllithium oder Alkylmagnesiumhalogenid, durch Alkyl ersetzen.

Die Ausgangsketale der Formel III lassen sich aus dem zugrundeliegenden Methylaryl-keton der Formel XIV

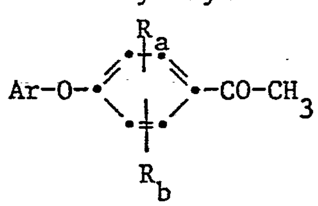

(XIV)

durch Reaktion mit dem gewünschten Diol in einem inerten Lösungsmittel z.B. einem halogenierten Kohlenwasserstoff (wie Methylenchlorid, Ethylenchlorid, Chloroform, Tetrachlorkohlenstoff usw.) und gleichzeitige oder anschliessende Halogenierung gewinnen. Zur Beschleunigung der Reaktion ist ein Zusatz von p-Toluolsulfonsäure vorteilhaft.

Die Ketone der Formel IV können durch Halogenierung der Ausgangsketone XIV zu XV

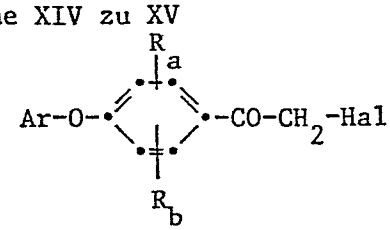

(XV)

und Weiterreaktion von XV, analog zu Variante A, mit einem Azol der Formel II hergestellt werden. Hierbei steht Hal bevorzugt für Chlor oder Brom.

Die Ketale III, VI, IX und X erhält man analog zu Variante B durch Reaktion des Ausgangsketons z.B. der Formel IV mit einem geeigneten Alkohol oder Diol.

Erhaltene freie Verbindungen der Formel I können in an sich bekannter Weise in Salze übergeführt werden, u.a. durch Behandeln mit einer Base oder mit einem geeigneten Salz einer Carbonsäure, üblicherweise in Gegenwart eines Lösungs- oder Verdünnungsmittels.

Erhaltene Salze können in an sich bekannter Weise in die freien Verbindungen umgewandelt werden, z.B. durch Behandeln mit einem sauren Reagens, wie einer Mineralsäure.

Die Verbindungen, inklusive ihre Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukt erhältlichen Verbindung ausgeht und die fehlenden Verfahrensschritte ausführt, oder einen Ausgangsstoff unter den Reaktionsbedingungen gebildet oder in Form eines Derivates davon, gegebenenfalls eines Salzes, verwendet.

Beim Verfahren der vorliegenden Erfindung werden vorzugsweise solche Ausgangsstoffe verwendet, welche zu den eingangs als besonders wertvoll bezeichneten Verbindungen führen. Neue Ausgangsstoffe und Verfahren zu ihrer Herstellung sind in der Erfindung inbegriffen.

Bei allen beschriebenen Ketalisierungs-Reaktionen eines Ketons mit einem substituierten α,β- oder α,γ-Diol entstehen vorwiegend Gemische von Diastereomeren des resultierenden Ketals. Entsprechend bilden sich aus den Ausgangsketonen im allgemeinen Diastereomerengemische der Endprodukte I. Die Verbindungen der Formel I können beispielsweise in nachfolgenden beiden diastereomeren Formen vorliegen:

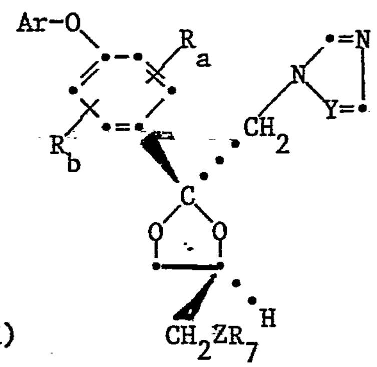

(XVI)

A-Typen

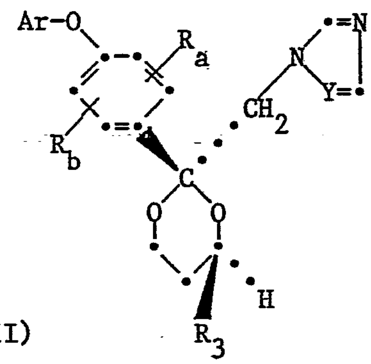

(XVII)

Die Konfiguration vom Typ A soll hier und im folgenden als das "trans"-Isomere bezeichnet werden.

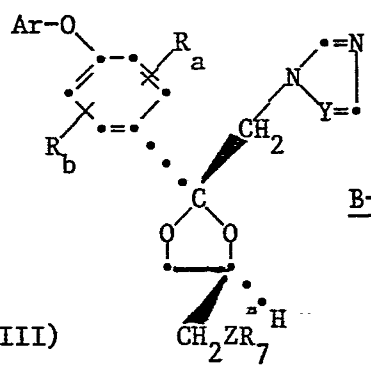

(XVIII)

B-Typen

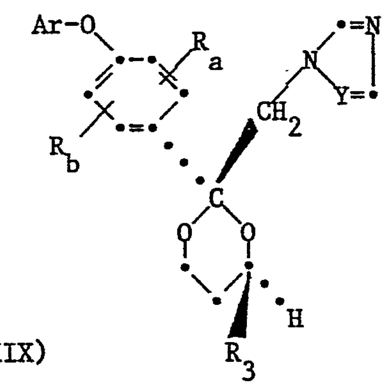

(XIX)

Die Symbole in den räumlich wiedergegebenen Strukturen sollen folgende Bedeutungen haben:

•••• = hinter

— = in

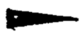 = vor der Zeichenebene.

Die Konfiguration vom Typ B soll entsprechend als das "cis"-Isomere bezeichnet werden. Die Trennung der beiden Diastereomeren kann

beispielsweise durch fraktionierte Kristallisation oder durch Chromatographie (Dünn-, Dickschicht-, Säulenchromatographie, Flüssigkeitshochdruckchromatographie usw.) erfolgen. Die beiden Isomeren zeigen unterschiedliche biologische Wirkungen. Im allgemeinen werden für praktische Zwecke die Diastereomerengemische verwendet.

Die Erfindung betrifft sämtliche isomeren Verbindungen der Formel I und ihre Salze.

1-(β-Aryl)-ethylimidazolylketale, worin Aryl für substituiertes Phenyl oder Naphthyl steht, werden in folgenden Referenzen als Fungizide und Bakterizide zitiert: US-PS: 3 575 999, 3 936 470, 4 101 664, 4 101 666, 4 156 008.

Die die Verwendbarkeit der Verbindungen der Formel I und ihrer pharmazeutisch verwendbaren Säureadditionssalze zur Bekämpfung an Warmblütern parasitierender Pilze begründenden antimycotischen Eigenschaften können beispielsweise in vitro mittels üblicher mikrobiologischer Untersuchungsverfahren, z.B. anhand ihrer toxischen Wirkung auf an Warmblütern parasitierende Pilzstämme, wie Trychophyton mentagryphites, Microsporum canis, Sporotrichum schenkii, Aspergillus fumigatus und Candida albicans, ebenso in vivo am Meerschweinchen anhand der Heilwirkung auf experimentelle Infestationen der Rückenhaut mit Trychophyton, z.B. T. rubrum, nach peroraler bzw. lokaler Applikation.

Die antikonvulsive Wirksamkeit der Verbindungen der Formel I und ihrer pharmazeutisch verwendbaren Säureadditionssalze kann in vivo beispielsweise an der Maus im Pentatetrazolkrampf-Test im Dosisbereich von etwa 10 bis etwa 100 mg/kg p.o., ebenso im Elektroschock-Test im Dosisbereich von etwa 10 bis etwa 100 mg/kg p.o. demonstriert werden. Die anxiolytische Wirksamkeit lässt sich beispielsweise an der Maus

und anderen Kleinnagern anhand des Gellert-Testes und des Quatre Plaques-Testes im Dosisbereich von etwa 10 bis etwa 100 mg/kg p.o. zeigen. Man kann auch auf eine signifikante antimanische Wirkung der neuen Verbindungen schliessen.

Die Erfindung betrifft dementsprechend ebenfalls die Verwendung von Verbindungen der Formel I und ihrer pharmazeutisch verwendbaren Säureadditionssalze zur topischen bzw. lokalen und systemischen Bekämpfung an Warmblütern parasitierender Pilze bzw. zur systemischen Behandlung von verschiedenen Formen der Epilepsie, von Angst- und Spannungszuständen und von manischen Gemütszuständen, insbesondere als Wirkstoff in bzw. zur Herstellung von pharmazeutischen Präparaten zur enteralen, parenteralen, topischen bzw. lokalen Applikation sowie derartige pharmazeutische Präparate.

Bei den erfindungsgemässen pharmazeutischen Präparaten, welche Verbindungen der Formel I oder pharmazeutisch verwendbare Salze davon enthalten, handelt es sich dementsprechend um solche zur enteralen, wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand sowie von der Applikationsweise ab.

Im Normalfall ist für einen etwa 75 kg schweren Warmblüter bei oraler Applikation eine ungefähre Tagesdosis von etwa 50-500 mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60% des Wirkstoffs. Erfindungsgemässe pharmazeutische Präparate zur eteralen bzw. parenteralen Verabreichung sind z.B. solche in Dosiseinheitsformen wie Dragées,

Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess-regulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln

aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyethylenglykole oder Paraffinkohlenwasserstoff in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat, oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20% des Wirkstoffs enthalten.

Creme sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukten davon, wie Polyglycerinfettsäureester oder Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cerylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe etc.

Salben sind Wasser-in-Oel-Emulsionen, die bis zu 70%, vorzugsweise jedoch von etwa 20% bis etwa 50% Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens wässrig-ethanolische Grundlage auf, der u.a. Polyalkohole, z.B. Glycerin, Glykole, und/oder Poly-Ethylenglykol, als Feuchthaltemittel zur Herabsetzung der Verdunstung, und rückfettende Substanzen, wie Fettsäureester mit niedrigen Poly-Ethylenglykolen, d.h. im wässrigen Gemisch lösliche, lipophile Substanzen als Ersatz für die der Haut mit dem Ethanol entzogene Fettsubstanzen, und, falls notwendig, andere Hilfs- und Zusatzmittel zugegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die nachfolgenden Beispiele dienen zur näheren Erläuterung der Erfindung, ohne dieselbe einzuschränken. Temperaturen sind in Celsiusgraden, Drucke in mbar angegeben. Prozente und Teile beziehen sich auf das Gewicht. RT bedeutet Raumtemperatur, h steht für Stunde.

Beispiel 1: Herstellung von

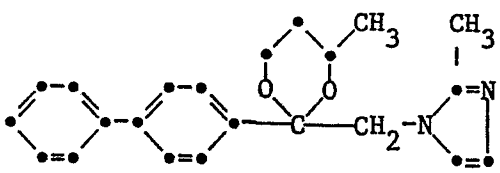

(1.3)

2-(p-Phenoxyphenyl)-2-[1-(2-methylimidazol)methyl]-4-methyl-1,3-dioxan

a) Zwischenprodukt

a) 2-(p-Phenoxyphenyl)-2-brommethyl-4-methyl-1,3-dioxan

10 Teile 2-(p-Phenoxyphenyl)-2-oxo-1-brommethan und 4 Teile 1,3-Butandiol werden in 40 ml absolutem Toluol in Gegenwart von 0,2 Teilen katalytisch wirkender p-Toluolsulfonsäure 3 Stunden unter Rückfluss erhitzt, wobei gebildetes Wasser mit einem Wasserabscheider abgetrennt wird. Nach dem Abkühlen auf Raumtemperatur wird das Reaktionsgemisch zweimal mit je 20 ml Wasser gewaschen, über Natriumsulfat getrocknet, filtriert, das Lösungsmittel verdampft und das Rohprodukt aus Isopropanol umkristallisiert. Farblose Kristalle. Smp. 96-106°.

b) Endprodukt

3,8 Teile 2-Methylimidazol-Natriumsalz und eine katalytisch wirkende Menge Kaliumjodid werden in 40 ml Dimethylformamid zusammen mit 10,2 Teilen des nach a) hergestellten 2-(p-Phenoxyphenyl)-2-brommethyl-4-methyl-1,3-dioxan 30 h bei einer Innentemperatur von +120° gerührt. Nach dem Abkühlen auf Raumtemperatur werden 300 ml Wasser zugegeben, dreimal mit je 30 ml Ethylacetat extrahiert, die vereinigten Extrakte zweimal mit je 20 ml Wasser gewaschen, über Natriumsulfat getrocknet, filtriert und das Lösungsmittel verdampft. Der ölige Rückstand wird säulenchromatographisch (Kieselgel/Ethylacetat) gereinigt. Das Laufmittel wird abgedampft. Man erhält eine viskose Masse mit dem $n_D^{22,5} = 1,5634$.

Beispiel 2: Herstellung

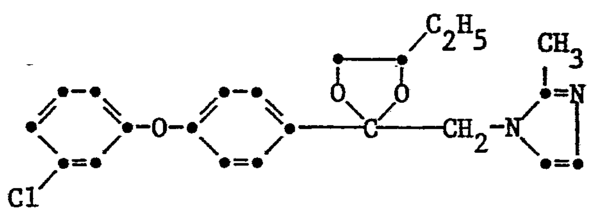

(1.50)

2-[p-(m-Chlorphenoxy)phenyl]-2-[1-(2-methylimidazolyl)methyl]-4-ethyl-1,3-dioxolan

1,4 Teile 2-Methylimidazol-Natriumsalz und eine katalytisch wirkende Menge Kaliumjodid werden in 50 ml Dimethylformamid zusammen mit 4 Teilen 2-[p-(3-Chlorphenoxy)phenyl]-2-brommethyl-4-ethyl-1,3-dioxolan 17 h bei einer Innentemperatur von 125° gerührt. Nach dem Abkühlen wird das braune Reaktionsgemisch mit 150 ml Wasser versetzt, dreimal mit je 50 ml Ethylacetat extrahiert, die vereinigten Extrakte zweimal mit je 50 ml Wasser gewaschen, über Natriumsulfat getrocknet, filtriert und das Lösungsmittel verdampft. Das ölige Rohprodukt wird über eine 35 cm lange Kieselgelsäule mittels Aceton/Ethylacetat (1:1) chromatographiert. Das Laufmittel wird verdampft. Man erhält eine viskose Masse mit dem $n_D^{22} = 1,5685$.

Beispiel 3: Herstellung von

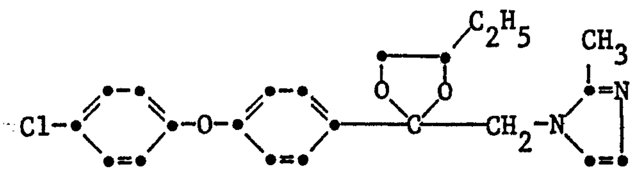

(1.2)

2-[p-(p-Chlorphenoxy)phenyl]-2-[1-(2-methylimidazolyl)methyl]-4-ethyl-1,3-dioxolan

a) Zwischenprodukte

α) 2-[p-(p-Chlorphenoxy)phenyl]-2-methyl-4-ethyl-1,3-dioxolan

37 Teile 4-(p-Chlorphenoxy)acetophenon und 18 Teile 1,2-Butandiol werden in 400 ml absolutem Toluol in Gegenwart von 2 Teilen katalytisch wirkender p-Toluolsulfonsäure 14 h am Wasserabscheider unter Rückfluss erhitzt. Nach dem Abkühlen auf RT wird das Reaktionsgemisch zweimal mit je 400 ml Wasser gewaschen, über Natriumsulfat getrocknet, filtriert, das Lösungsmittel verdampft und das Rohprodukt zur Reinigung über eine 1 m lange Kieselgelsäule mittels Ligroin/Hexan/Ethylacetat/Toluol (5:3:1:1) chromatographiert. Das Produkt wird als leicht gelbliches Oel erhalten. $n_D^{22}$: 1,5527.

β) 2-[p-(p-Chlorphenoxy)phenyl]-2-brommethyl-4-ethyl-1,3-dioxolan

36,8 Teile des unter α) hergestellten 2-[p-(p-Chlorphenoxy)phenyl]-2-methyl-4-ethyl-1,3-dioxolans werden in 350 ml Chloroform zum Sieden erhitzt. Unter Beleuchtung mittels einer 150 Watt Spotlampe werden 19,4 Teile Brom, gelöst in 50 ml Chloroform, zugetropft und anschliessend 2 h unter Rückfluss erhitzt. Nach dem Abkühlen auf RT wird das Reaktionsgemisch zweimal mit je 200 ml Wasser gewaschen, über Natriumsulfat getrocknet, filtriert und am Wasserstrahlvakuum das Lösungsmittel abgezogen. Zur Reinigung wird das Rohprodukt über eine 1 m lange Kieselgelsäule mittels Toluol chromatographiert. Das Produkt wird als gelbes Oel mit dem $n_D^{23}$ = 1,5805 erhalten.

b) Endprodukt

5,0 Teile 2-Methylimidazol-Natriumsalz und eine katalytisch wirkende Menge Kaliumjodid werden in 80 ml Dimethylformamid zusammen mit 14,7 Teilen des nach β) hergestellten 2-[p-(p-Chlorphenoxy)phenyl]-2-brommethyl-4-ethyl-1,3-dioxolan 17 h bei einer Badtemperatur von 125°C gerührt. Nach dem Abkühlen auf Raumtemperatur wird das Reaktionsgemisch auf 600 ml Wasser gegossen, dreimal mit je 200 ml Ethylacetat extrahiert, die vereinigten organischen Phasen zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Aceton/Ethylacetat (1:1) chromatographiert. Nach Verdampfen des Laufmittels wird das Produkt als braunes Oel mit dem $n_D^{23}$ = 1,5721 erhalten.

Beispiel 4: Herstellung von

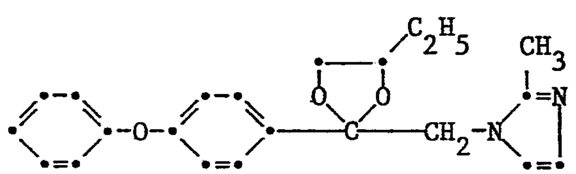

(1.1)

2-(p-Phenoxyphenyl)-2-[1-(2-methylimidazolyl)methyl]-4-ethyl-1,3-dioxolan

17 Teile 2-(p-Phenoxyphenyl)-3-brommethyl-4-ethyl-1,3-dioxolan, 8,4 Teile Kaliumcarbonat, 5,0 Teile 2-Methylimidazol und eine katalytisch wirkende Menge Natriumjodid werden in 100 ml Dimethylsulfoxid 24 h bei einer Innentemperatur von 125° gerührt. Nach dem Abkühlen auf RT wird das Reaktionsgemisch auf 600 ml Wasser gegossen, dreimal mit je 200 ml Ethylacetat extrahiert, die vereinigten Extrakte zweimal mit je 200 ml Wasser gewaschen, über Natriumsulfat getrocknet, filtriert und das Lösungsmittel verdampft. Man erhält eine viskose Masse mit dem $n_D^{22,5}$ = 1,5562.

Beispiel 5: Herstellung von

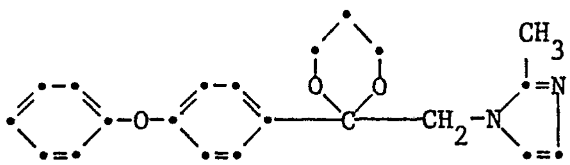

(1.17)

2-(p-Phenoxyphenyl)-2-[1-(2-methylimidazolyl)methyl]-1,3-dioxan

14 Teile 2-(p-Phenoxyphenyl)-2-brommethyl-1,3-dioxan, 7,2 Teile Kaliumcarbonat, 4,2 Teile 2-Methylimidazol und eine katalytisch wirkende Menge Kaliumjodid werden in 100 ml Dimethylsulfoxid 20 h bei einer Innentemperatur von 140° gerührt. Nach dem Abkühlen auf RT werden 600 ml Wasser zugegeben, dreimal mit je 200 ml Ether extrahiert, die vereinigten Extrakte zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Chloroform/Ether (1:1) chromatographiert. Das Laufmittel wird verdampft. Man erhält eine viskose Masse mit dem $n_D^{23}$ = 1,5632.

Beispiel 6: Herstellung von

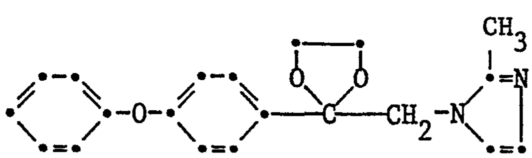

(1.12)

2-(p-Phenoxyphenyl)-2-[1-(2-methylimidazolyl)methyl]-1,3-dioxolan

4,5 Teile 2-(p-Phenoxyphenyl)-2-brommethyl-4-hydroxymethyl-1,3-dioxolan, 2,2 Teile Kaliumcarbonat, 1,3 Teile 2-Methylimidazol und eine katalytisch wirkende Menge Kaliumjodid werden in 50 ml Dimethylsulfoxid 4 h bei einer Innentemperatur von 140° gerührt. Nach dem Abkühlen auf RT werden 600 ml Wasser zugegeben, zweimal mit je 200 ml Ethylacetat extrahiert, die vereinigten Extrakte zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Aceton chromatographiert. Nach dem Verdampfen des Laufmittels kristallisiert der ölige Rückstand nach Zugabe von Petrolether. Beige Kristalle vom Smp. 166 - 170°.

Beispiel 7: Herstellung von

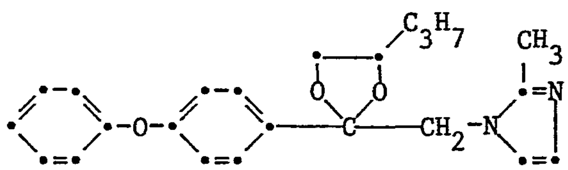

(1.29)

2-(p-Phenoxyphenyl)-2-[1-(2-methylimidazolyl)methyl]-4-n-propyl-1,3-dioxolan

10,3 Teile des Nitrats vom 1-(p-Phenoxyphenyl)-2-[1-(2-methylimidazolyl)]-ethanon, 6,1 Teile 1,2-Pentandiol, 6,9 Teile p-Toluolsulfonsäure, 20 Teile 1-Pentanol und 200 Teile Xylol werden 6 Tage am Wasserabscheider unter Rückfluss erhitzt und nach dem Abkühlen auf RT zweimal mit je 200 ml verdünnter Natronlauge und zweimal mit je 200 ml Wasser gewaschen. Die organische Phase wird über Natriumsulfat getrocknet, filtriert und das Lösungsmittel verdampft. Der ölige Rückstand wird über eine 1 m lange Kieselgelsäule mittels Ethylacetat chromatographiert. Nach dem Verdampfen des Laufmittels bleibt ein gelbliches Oel mit dem $n_D^{22,5}$ = 1,5565 zurück.

Beispiel 8: Herstellung von

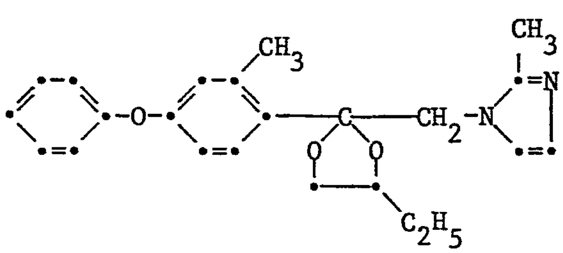

(1.7)

2-(2-Methyl-4-phenoxy-phenyl)-2-[1-(2-methylimidazolyl)methyl]-4-ethyl-1,3-dioxolan

a) Zwischenprodukte

α) 2-Methyl-4-phenoxy-phenacylbromid

36,6 Teile 2-Methyl-4-phenoxy-acetophenon werden in 160 ml Essigsäure gelöst auf 35° erwärmt und unter Rühren innerhalb von 1,5 h tropfenweise mit 25,9 Teilen Brom versetzt. Man lässt 1 h nachrühren, giesst in 1000 ml Eiswasser und schüttelt zweimal mit je 100 ml Diethylether aus. Die vereinigten Auszüge werden mit Wasser gewaschen, über Natriumsulfat getrocknet, filtriert und eingedampft. Der ölige Rückstand kristallisiert aus Hexan zu bräunlichen Kristallen vom Smp. 60 - 61°.

β) 2-(2-Methyl-4-phenoxy-phenyl)-2-brommethyl-4-ethyl-1,3-dioxolan

85,4 Teile 2-Methyl-4-phenoxy-phenacylbromid und 25,2 Teile Butan-1,2-diol werden in 250 ml Toluol gelöst. Man fügt 1 Teil p-Toluolsulfonsäure hinzu und erhitzt am Wasserabscheider 25 h zum Sieden. Man lässt auf RT abkühlen, wäscht dreimal mit je 250 ml Wasser, trocknet über Natriumsulfat und filtriert. Das Lösungsmittel wird unter vermindertem Druck abgedampft. Es bleibt ein rotbraunes Oel zurück.

b) Endprodukt

9,5 Teile 2-(2-Methyl-4-phenoxyphenyl)-2-brommethyl-4-ethyl-1,3-dioxolan gemäss β), 2,9 Teile 2-Methylimidazol und 4 Teile Kaliumtertiärbutanolat werden in 50 ml Dimethylsulfoxid 30 h bei 110° gerührt. Man lässt auf RT abkühlen, verdünnt mit 300 ml Wasser und schüttelt dreimal mit je 150 ml Essigester aus. Die Essigesterauszüge werden vereinigt, mit Wasser neutral gewaschen, über Natriumsulfat getrocknet, filtriert und zur Trockne eingedampft. Das Rohprodukt wird an ein Silicagelsäule (50 cm lang) mit Essigester als Laufmittel gereinigt. Nach Verdampfen desselben bleibt eine viskose Masse mit dem $n_D^{22,5}$ = 1,5640 zurück.

Beispiel 9: Herstellung von 2-[p-(m-Chlorphenoxy)phenyl]-2-[1-(2-methylimidazolyl)methyl]-4-ethyl-1,3-dioxolan-hydrochlorid

9,8 Teile 2-[p-(m-Chlorphenoxy)phenyl]-2-[1-(2-methylimidazolyl)methyl]-4-ethyl-1,3-dioxolan werden in 80 ml Diethylether gelöst und unter

Rühren tropfenweise mit 2,5 Teile konzentrierter Salzsäure versetzt. Der gebildete gelbliche Niederschlag wird nach 1 h abfiltriert, mit eiskaltem Diethylether gewaschen und getrocknet: Weisse Kristalle vom Smp. 158 - 162°.

Beispiel 10: Auf analoge Weise lassen sich auch die in der nachfolgenden Tabelle (Tab. I) beschriebenen Endprodukte (falls nicht besonders vermerkt Diastereomerengemische mit unterschiedlichen Mischungsverhältnissen) der Formel I herstellen:

Tabelle 1: Verbindungen der Formel

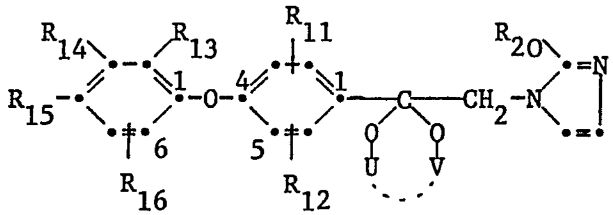

(Ia)

| Verb. Nr. | $R_{11}$ | $R_{12}$ | $R_{13}$ | $R_{14}$ | $R_{15}$ | $R_{16}$ | U.....V | $R_{20}$ | Salz | Physikal. Kenndaten (0°C) |
|---|---|---|---|---|---|---|---|---|---|---|
| 1.1 | H | H | H | H | H | H | 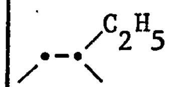 | $CH_3$ | - | viskose Masse, $n_D^{22,5}$ 1,5562 |
| 1.2 | H | H | H | H | Cl | H | 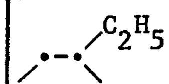 | $CH_3$ | - | viskose Masse, $n_D^{23}$ 1,5721 |
| 1.3 | H | H | H | H | H | H | 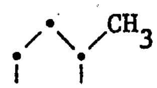 | $CH_3$ | - | viskose Masse, $n_D^{22,5}$ 1,5634 |
| 1.4 | H | H | H | Cl | Cl | H | 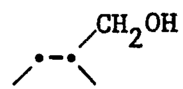 | $CH_3$ | - | |
| 1.5 | H | H | H | Cl | Cl | H | 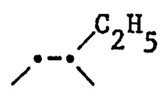 | $CH_3$ | - | viskose Masse, |
| 1.6 | H | H | H | Cl | Cl | H | 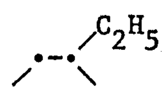 | $C_2H_5$ | - | |
| 1.7 | 2-$CH_3$ | H | H | H | H | H | 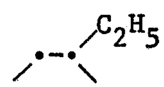 | $CH_3$ | - | viskose Masse, $n_D^{22,5}$ 1,5640 |
| 1.8 | 2-$CH_3$ | H | H | H | Cl | H | 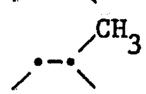 | $CH_3$ | - | viskose Masse, $n_D^{21}$ 1,5767 |

Tabelle 1: (Fortsetzung)

| Verb. Nr. | $R_{11}$ | $R_{12}$ | $R_{13}$ | $R_{14}$ | $R_{15}$ | $R_{16}$ | U.....V | $R_{20}$ | Salz | Physikal. Kenndaten (0°C) |
|---|---|---|---|---|---|---|---|---|---|---|
| 1.9 | 2-$CH_3$ | H | H | H | Cl | H | 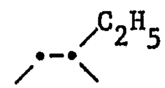 | $CH_3$ | - | Oel, $n_D^{21}$1,5711 |
| 1.10 | H | H | H | Cl | H | H | 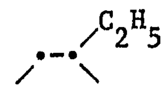 | $CH_3$ | - | viskose Masse, $n_D^{22}$1,5685 |
| 1.11 | 2-$CH_3$ | H | H | H | F | H | 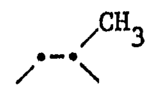 | $CH_3$ | - | |
| 1.12 | H | H | H | H | H | H | 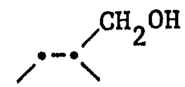 | $CH_3$ | - | Smp. 166 - 170° |
| 1.13 | H | H | H | H | Cl | H | 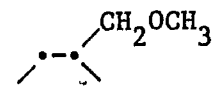 | $CH_3$ | - | |
| 1.14 | H | H | H | H | H | H | 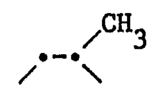 | $CH_3$ | - | |
| 1.15 | 2-Cl | H | H | H | H | H | 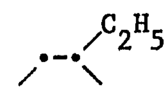 | $CH_3$ | - | Oel, $n_D^{21}$1.5708 |
| 1.16 | 2-Cl | H | H | H | Cl | H | 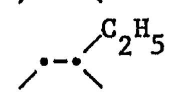 | $CH_3$ | - | |
| 1.17 | H | H | H | H | H | H | 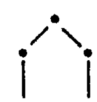 | $CH_3$ | - | viskose Masse, $n_D^{23}$1,5632 |

Tabelle 1: (Fortsetzung)

| Verb. Nr. | $R_{11}$ | $R_{12}$ | $R_{13}$ | $R_{14}$ | $R_{15}$ | $R_{16}$ | U.....V | $R_{20}$ | Salz | Physikal. Kenndaten (O°C) |
|---|---|---|---|---|---|---|---|---|---|---|
| 1.18 | H | H | H | H | F | H | 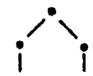 | $CH_3$ | - | |
| 1.19 | 2-$CH_3$ | 6-$CH_3$ | H | H | H | H | 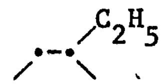 | $CH_3$ | - | |
| 1.20 | H | H | $CH_3$ | H | Cl | H | 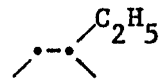 | $CH_3$ | - | |
| 1.21 | H | H | H | H | Cl | H | 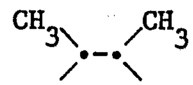 | $CH_3$ | - | |
| 1.22 | H | H | H | H | F | H | 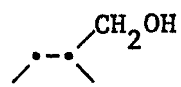 | $CH_3$ | - | |
| 1.23 | 2-$CH_3$ | H | H | H | F | H | 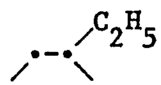 | $CH_3$ | - | |
| 1.24 | H | H | H | H | Cl | H | 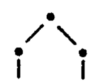 | $CH_3$ | - | Smp. 88 - 90° |
| 1.25 | 2-$CH_3$ | H | H | H | F | H | 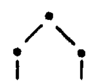 | $CH_3$ | - | |
| 1.26 | 2-$CH_3$ | H | H | H | Br | H | 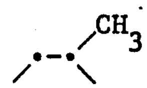 | $CH_3$ | - | |

Tabelle 1: (Fortsetzung)

| Verb. Nr. | $R_{11}$ | $R_{12}$ | $R_{13}$ | $R_{14}$ | $R_{15}$ | $R_{16}$ | U.....V | $R_{20}$ | Salz | Physikal. Kenndaten (0°C) |
|---|---|---|---|---|---|---|---|---|---|---|
| 1.27 | 2-$CH_3$ | H | H | H | Cl | H | 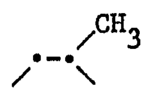 | $CH_3$ | $HNO_3$ | Smp. 181-183° |
| 1.28 | H | H | H | $CH_3$ | Cl | H | 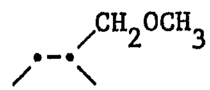 | $CH_3$ | - | Oel, $n_D^{22}$ 1,5599 |
| 1.29 | H | H | H | H | H | H | 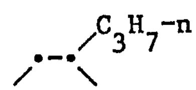 | $CH_3$ | - | Oel, $n_D^{22,5}$ 1,5565 |
| 1.30 | H | H | Cl | H | Cl | H | 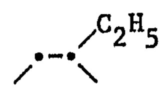 | $CH_3$ | - | Oel, $n_D^{22,5}$ 1,5732 |
| 1.31 | H | H | H | H | Cl | H | 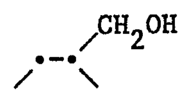 | $CH_3$ | - | |
| 1.32 | 2-Cl | H | H | H | F | H | 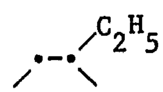 | $CH_3$ | - | |
| 1.33 | H | H | H | Cl | H | H | 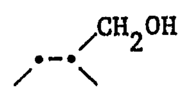 | $CH_3$ | - | |
| 1.34 | 2-$CH_3$ | H | H | Cl | H | H | 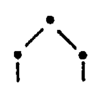 | $CH_3$ | - | |
| 1.35 | 2-$CH_3$ | 5-$CH_3$ | H | Cl | H | H | 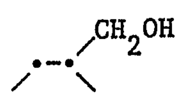 | $CH_3$ | - | |

Tabelle 1: (Fortsetzung)

| Verb. Nr. | $R_{11}$ | $R_{12}$ | $R_{13}$ | $R_{14}$ | $R_{15}$ | $R_{16}$ | U.....V | $R_{20}$ | Salz | Physikal. Kenndaten (0°C) |
|---|---|---|---|---|---|---|---|---|---|---|
| 1.36 | 2-$CH_3$ | H | H | H | H | H | 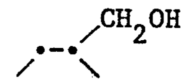 | $CH_3$ | - | |
| 1.37 | 2-$CH_3$ | 5-$CH_3$ | H | H | H | H | 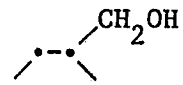 | $CH_3$ | - | |
| 1.38 | 2-$CH_3$ | 5-$CH_3$ | H | H | H | H | 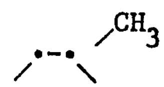 | $CH_3$ | - | |
| 1.39 | 2-$CH_3$ | 5-$CH_3$ | H | H | Cl | H | 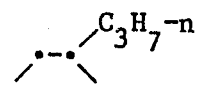 | $C_2H_5$ | HCl | |
| 1.40 | 2-$C_2H_5$ | H | H | H | Cl | H | 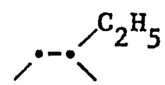 | $CH_3$ | - | $n_D^{24,5}$ 1,5678 |
| 1.41 | 2-$CH_3$ | H | H | H | H | H | 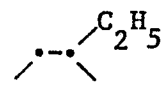 | $CH_3$ | $HNO_3$ | Smp. 124-126° |
| 1.42 | H | H | Cl | H | H | H | 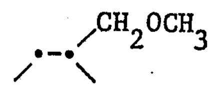 | $CH_3$ | - | $n_D^{22}$ 1,5682 |
| 1.43 | H | H | Cl | H | H | H | 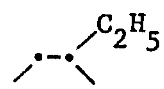 | $CH_3$ | - | Oel, $n_D^{22}$ 1,5690 |
| 1.44 | H | H | H | Cl | H | H | 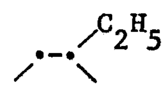 | $CH_3$ | HCl | Smp. 158-162° |

Tabelle 1: (Fortsetzung)

| Verb. Nr. | $R_{11}$ | $R_{12}$ | $R_{13}$ | $R_{14}$ | $R_{15}$ | $R_{16}$ | U.....V | $R_{20}$ | Salz | Physikal. Kenndaten (0°C) |
|---|---|---|---|---|---|---|---|---|---|---|
| 1.45 | H | H | H | H | H | H | 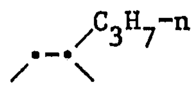 | $C_2H_5$ | - | Oel, $n_D^{24}$ 1.5596 |
| 1.46 | H | H | H | $CH_3$ | Cl | H | 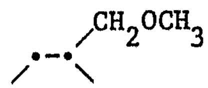 | $C_2H_5$ | - | |
| 1.47 | H | H | H | H | H | H | 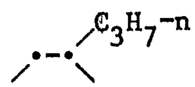 | $C_3H_7$-n | - | Oel, $n_D^{23,5}$ 1,5631 |
| 1.48 | H | H | H | H | H | H | 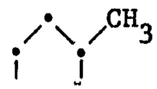 | $C_2H_5$ | - | viskose Masse, $n_D^{23}$ 1,5638 |
| 1.49 | H | H | H | $CH_3$ | Cl | H | 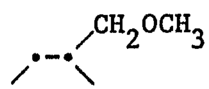 | $C_3H_7$-i | - | |
| 1.50 | H | H | Cl | H | H | H | 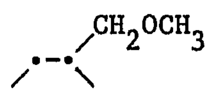 | $C_3H_7$-i | - | |
| 1.51 | H | H | H | H | H | H | 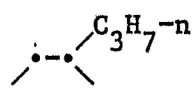 | $C_3H_7$-i | - | |
| 1.52 | H | H | H | H | H | H | 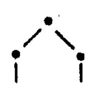 | $C_2H_5$ | - | |
| 1.53 | H | H | H | H | H | H | 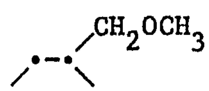 | $C_4H_9$-n | - | |

Tabelle 1: (Fortsetzung)

| Verb. Nr. | $R_{11}$ | $R_{12}$ | $R_{13}$ | $R_{14}$ | $R_{15}$ | $R_{16}$ | U.....V | $R_{20}$ | Salz | Physikal. Kenndaten (0°C) |
|---|---|---|---|---|---|---|---|---|---|---|
| 1.54 | H | H | H | H | H | H | 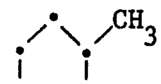 | $C_3H_7$-i | - | |
| 1.55 | H | H | H | H | H | H | 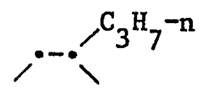 | 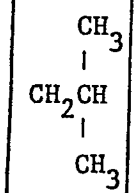 | - | |
| 1.56 | H | H | Cl | H | H | H | 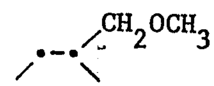 | $C_2H_5$ | - | |

Beispiel 11: Gelatinekapseln, enthaltend 200 mg [p-(p-Chorphenoxy)-phenyl]-2-[1-(2-methylimidazolyl)methyl-4-ethyl-1,3-dioxolan als Wirkstoff, können z.B. wie folgt hergestellt werden:

| Zusammensetzung (für 1000 Kapseln) | |
|---|---|
| Wirkstoff | 100 g |
| Laktose, gemahlen | 100 g |

Der Wirkstoff und die Laktose (feinst gemahlen) werden gut miteinander vermischt. Das erhaltene Pulver wird gesiebt und in Portionen zu je 0,20 g in Gelatinekapseln abgefüllt.

Beispiel 12: Tabletten enthaltend 25 mg Wirkstoff, z.B. 2-[p-(p-Chlorphenoxy)phenyl]-2-[1-(2-methylimidazolylmethyl]-4-ethyl-1,3-dioxolan, können folgendermassen erhalten werden:

| Bestandteile (für 1000 Tabletten) | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben

und zu beidseitig konkaven Tabletten von etwa 6 mm Durchmesser verpresst.

Beispiel 13: Tabletten enthaltend 75 mg Wirkstoff, z.B. 2-[p-(p-Chlorphenoxy)phenyl]-2-[1-(2-methylimidazolyl)methyl]-4-ethyl-1,3-dioxolan, können folgendermassen hergestellt werden:

| Bestandteile (für 1000 Tabletten) | |
|---|---|
| Wirkstoff | 75,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 14: In analoger Weise wie in den Beispielen 11 bis 13 beschrieben kann man auch pharmazeutische Präparate, enthaltend eine andere der gemäss Tabelle 1 erhältlichen Verbindungen, herstellen.

Patentansprüche für die Vertragsstaaten: BE, CH, LI, DE, FR, GB, IT, LU, NL, SE

1. Neue Arylphenylether-derivate der Formel I

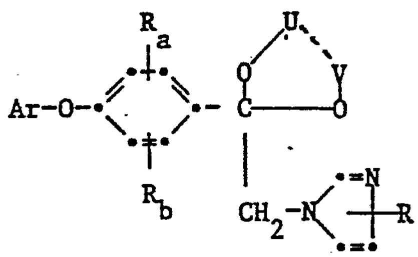

(I) ,

worin

R für $C_1$-$C_6$-Alkyl steht;

$R_a$ und $R_b$ unabhängig voneinander Wasserstoff, Halogen oder $C_1$-$C_3$-Alkyl bedeuten;

Ar unsubstituiertes oder durch Halogen, $C_1$-$C_3$-Alkyl, $C_1$-$C_3$-Alkoxy und/oder Trifluormethyl ein- oder mehrfach substituiertes Phenyl bedeutet;

U und V unabhängig voneinander für gegebenenfalls durch Halogen oder $C_1$-$C_6$-Alkoxy substituiertes $C_1$-$C_{12}$-Alkyl stehen oder zusammen eine der folgenden Alkylenbrücken

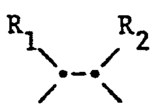 , 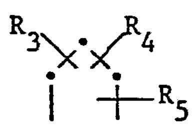 oder 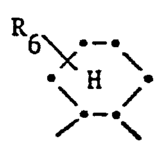 bilden, wobei

$R_1$ und $R_2$ unabhängig voneinander für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_3$-Alkyl substituiertes Phenyl oder für die Gruppe $-CH_2-Z-R_7$ stehen, wobei

Z Sauerstoff oder Schwefel bedeutet und

$R_7$ für Wasserstoff, $C_1$-$C_8$-Alkyl, ein durch $C_1$-$C_2$-Alkoxy substituiertes $C_1$-$C_8$-Alkyl, $C_3$-$C_4$-Alkenyl, 2-Propinyl, 3-Halogen-2-propinyl, Phenyl, ein durch Halogen, $C_1$-$C_3$-Alkyl, $C_1$-$C_3$-Alkoxy, Nitro und/oder $CF_3$ ein- oder mehrfach substituiertes Phenyl, Benzyl oder ein durch

Halogen, $C_1$-$C_3$-Alkyl und/oder $C_1$-$C_3$-Alkoxy ein- oder mehrfach substituiertes Benzyl steht;

$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$-$C_3$-Alkyl steht;

unter Einschluss ihrer Säureadditionssalze.

2\. Verbindungen gemäss Anspruch 1, worin R, $R_a$ und $R_b$ die in Anspruch 1 angegebenen Bedeutungen haben, Ar eine Gruppe der Formel

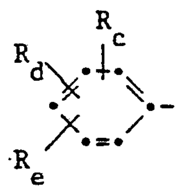

darstellt, in der $R_c$, $R_d$ und $R_e$ unabhängig voneinander Wasserstoff, Halogen, Trifluormethyl, $C_1$-$C_3$-Alkyl oder $C_1$-$C_3$-Alkoxy bedeuten, und U und V unabhängig voneinander $C_1$-$C_6$-Alkyl oder durch Halogen bzw. $C_1$-$C_2$-Alkoxy substituiertes $C_2$-$C_4$-Alkyl oder gemeinsam eine der folgenden Alkylengruppen

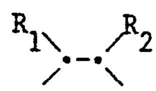 oder 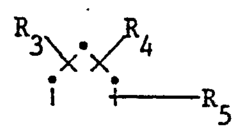

darstellen, worin $R_1$, $R_2$, $R_3$, $R_4$ und $R_5$ unabhängig voneinander Wasserstoff oder $C_1$-$C_4$-Alkyl, wobei die Gesamtzahl der Kohlenstoffatome von $R_3$, $R_4$ und $R_5$ 6 nicht übersteigt, oder $R_1$ Wasserstoff und $R_2$ eine Gruppe der Formel $CH_2OR_7$ darstellt, in der $R_7$ $C_1$-$C_4$-Alkyl, durch $C_1$-$C_2$-Alkoxy substituiertes $C_2$-$C_4$-Alkyl, $C_3$-$C_4$-Alkenyl oder Prop-2-inyl bedeutet, unter Einschluss ihrer Säureadditionssalze.

3\. Verbindungen gemäss Anspruch 1, worin R die unter der Formel I angegebene Bedeutung hat, $R_a$ und $R_b$ unabhängig voneinander Wasserstoff, Methyl, Chlor oder Brom bedeuten, Ar unsubstituiertes oder durch Halogen, Methyl oder Trifluormethyl substituiertes Phenyl bedeutet und U und V unabhängig voneinander

$C_1$-$C_3$-Alkyl, gegebenenfalls durch Halogen oder $C_1$-$C_2$-Alkoxy substituiertes $C_2$-$C_3$-Alkyl oder zusammen eine der folgenden Alkylengruppen

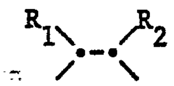

oder

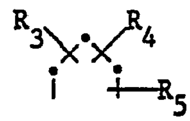

bilden, worin $R_1$, $R_2$, $R_3$, $R_4$ und $R_5$ unabhängig voneinander für Wasserstoff oder $C_1$-$C_4$-Alkyl stehen, wobei die Gesamtzahl der Kohlenstoffatome in $R_3$, $R_4$ und $R_5$ die Zahl 4 nicht übersteigen sollen, und ihre Säureadditionssalze.

4. Verbindungen gemäss Anspruch 1, worin R die unter der Formel I angegebene Bedeutung hat, $R_a$ und $R_b$ Wasserstoff bedeuten, Ar für unsubstituiertes oder durch Halogen oder Methyl substituiertes Phenyl und U sowie V gemeinsam eine Gruppe der Formel

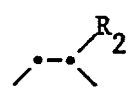

oder

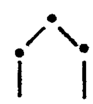

darstellen, in der $R_2$ $C_1$-$C_4$-Alkyl, Hydroxy-$C_1$-$C_3$-alkyl oder $C_1$-$C_2$-Alkoxy-$C_1$-$C_2$-alkyl bedeutet, und ihre Säureadditionssalze.

5. 2-[p-(p-Chlorphenoxy)phenyl]-2-[1-(2-methylimidazolyl)methyl]-4-ethyl-1,3-dioxolan gemäss Anspruch 1 oder

2-[p-(2,4-Dichlorphenoxy)phenyl]-2-[1-(2-methylimidazolyl)methyl]-4-ethyl-1,3-dioxolan gemäss Anspruch 1 oder ein Säureadditionssalz davon.

6. 2-(p-Phenoxyphenyl)-2-[1-(2-methylimidazolyl)methyl-4-methyl-1,3-dioxan, 2-[4-(p-chlorphenoxy)-2-methyl-phenyl]-2-[1-(2-methylimidazolyl)methyl]-4-methyl-1,3-dioxolan, 2-[4-(p-chlorphenoxy)-2-methylphenyl]-2-(1-(2-methylimidazolyl)methyl]-4-ethyl-1,3-dioxolan, 2-[p-(m-chlorphenoxy)phenyl]-2-[1-(2-methylimidazolyl)methyl]-4-ethyl-1,3-dioxolan, 2-(2-chlor-4-phenoxy-phenyl)-2-[1-(2-methylimidazolyl)-methyl]-4-ethyldioxolan, 2-[p-chlorphenoxy)phenyl]-2-[1-(2-methyl-

imidazolyl)methyl-1,3-dioxan, 2-[p-(4-chlor-3-methyl-phenoxy)-phenyl]-2-[1-(2-methylimidazolyl)methyl]-4-methoxymethyl-1,3-dioxolan oder 2-(p-phenoxyphenyl)-2-[1-(2-methylimidazolyl)-methyl]-4-propyl-dioxolan gemäss Anspruch 1 oder jeweils ein Säureadditionssalz davon.

7. Eine Verbindung gemäss einem der Ansprüche 1 - 6 zur Anwendung in einem Verfahren zur therapeutischen Behandlung des menschlichen oder tierischen Körpers.

8. Eine Verbindung gemäss einem der Ansprüche 1 - 6 zur Anwendung gemäss Anspruch 7 als Antikonvulsivum und/oder Anxiolytikum.

9. Pharmazeutische Präparate enthaltend mindestens eine Verbindung gemäss einem der Ansprüche 1 - 8 gegebenenfalls in Form eines pharmazeutisch verwendbaren Säureadditionssalzes neben üblichen pharmazeutischen Hilfsstoffen.

10. Verfahren zur Herstellung neuer Arylphenylether-derivate der Formel I

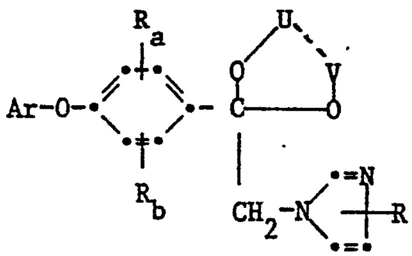

(I) ,

worin

R für $C_1$-$C_6$-Alkyl steht;

$R_a$ und $R_b$ unabhängig voneinander Wasserstoff, Halogen oder $C_1$-$C_3$-Alkyl bedeuten;

Ar unsubstituiertes oder durch Halogen, $C_1$-$C_3$-Alkyl, $C_1$-$C_3$-Alkoxy und/oder Trifluormethyl ein- oder mehrfach substituiertes Phenyl bedeutet;

U und V unabhängig voneinander für gegebenenfalls durch Halogen oder $C_1$-$C_6$-Alkoxy substituiertes $C_1$-$C_{12}$-Alkyl stehen oder zusammen eine der folgenden Alkylenbrücken

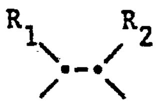 , 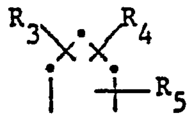 oder 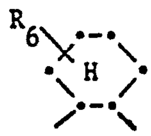 bilden, wobei

$R_1$ und $R_2$ unabhängig voneinander für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_3$-Alkyl substituiertes Phenyl oder für die Gruppe $-CH_2-Z-R_7$ stehen, wobei

Z Sauerstoff oder Schwefel bedeutet und

$R_7$ für Wasserstoff, $C_1$-$C_8$-Alkyl, ein durch $C_1$-$C_2$-Alkoxy substituiertes $C_1$-$C_8$-Alkyl, $C_3$-$C_4$-Alkenyl, 2-Propinyl, 3-Halogen-2-propinyl, Phenyl, ein durch Halogen, $C_1$-$C_3$-Alkyl, $C_1$-$C_3$-Alkoxy, Nitro und/oder $CF_3$ ein- oder mehrfach substituiertes Phenyl, Benzyl oder ein durch Halogen, $C_1$-$C_3$-Alkyl und/oder $C_1$-$C_3$-Alkoxy ein- oder mehrfach substituiertes Benzyl steht;

$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$-$C_3$-Alkyl steht;

unter Einschluss ihrer Säureadditionssalze, dadurch gekennzeichnet, dass man

A) eine Verbindung der Formel II

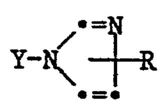 (II) ,

worin Y Wasserstoff oder ein Metallkation darstellt, mit einer Verbindung der Formel III

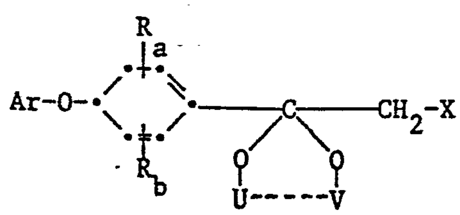 (III),

worin X eine nukleofuge Abgangsgruppe bedeutet, kondensiert oder

B) in einer Verbindung der Formel IV

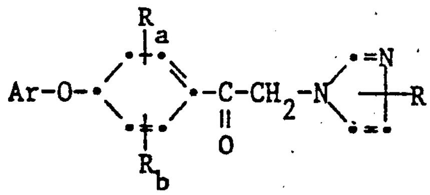 (IV)

die Carbonylgruppe in eine Gruppe der Formel V

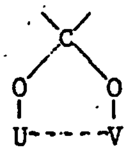 (V)

überführt, oder

C) zur Herstellung von Verbindungen der Formel I, worin U und V gemeinsam eine Gruppe der Formel $-CH_2-CH(CH_2ZR_7')-$ darstellen und $R_7'$ einen von Wasserstoff verschiedenen Rest $R_7$ bedeutet, Verbindungen der Formeln VI und VII

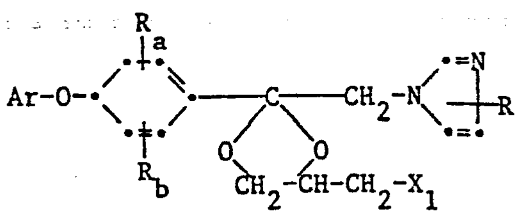 (VI) und $X_2 - R_7$ (VII),

worin einer der Reste $X_1$ und $X_2$ gegebenenfalls in Salzform vorliegendes Hydroxy oder Mercapto, z.B. der Formel -Z-Y, in der Y Wasserstoff oder vorzugsweise ein Metallkation ist, und der andere eine nukleofuge Abgangsgruppe X bedeutet oder sowohl $X_1$ als auch $X_2$ Hydroxygruppen darstellen, miteinander kondensiert oder

D) Verbindungen der Formeln VIII und IX

$Ar-X_3$ (VIII) und 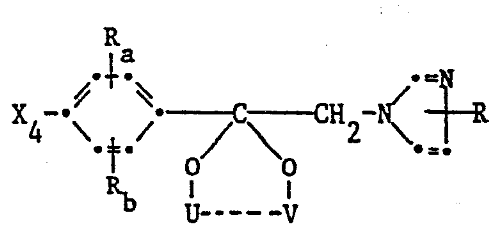 (IX),

worin einer der Reste $X_3$ und $X_4$ eine Gruppe -O-Y, in der Y Wasserstoff oder vorzugsweise ein Metallkation ist, und der andere einen

gegen Aryloxy austauschbaren Rest bedeutet, miteinander kondensiert oder

E) eine Verbindung der Formel

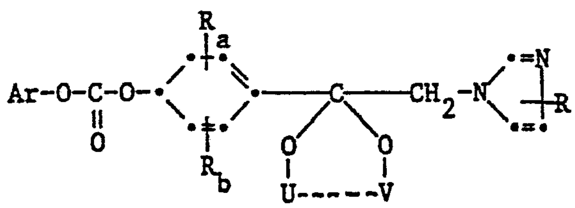 (X)

intramolekular decarboxyliert und, wenn erwünscht, eine verfahrensgemäss erhältliche Verbindung in eine andere Verbindung der Formel I umwandelt und/oder eine verfahrensgemäss erhältliche freie Verbindung in ein Säureadditionssalz, ein verfahrensgemäss erhältliches Säureadditionssalz in die freie Verbindung oder in ein anderes Säureadditionssalz überführt.

11. Die im Verfahren gemäss Anspruch 10 verwendeten neuen Ausgangsstoffe, gebildeten neuen Zwischenprodukte und erhältlichen Endstoffe.

0112284

Patentansprüche für den Vertragsstaat AT

1. Verfahren zur Herstellung neuer Arylphenylether-derivate der Formel I

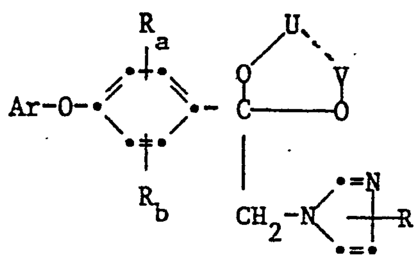

(I) ,

worin

R für $C_1$-$C_6$-Alkyl steht;

$R_a$ und $R_b$ unabhängig voneinander Wasserstoff, Halogen oder $C_1$-$C_3$-Alkyl bedeuten;

Ar unsubstituiertes oder durch Halogen, $C_1$-$C_3$-Alkyl, $C_1$-$C_3$-Alkoxy und/oder Trifluormethyl ein- oder mehrfach substituiertes Phenyl bedeutet;

U und V unabhängig voneinander für gegebenenfalls durch Halogen oder $C_1$-$C_6$-Alkoxy substituiertes $C_1$-$C_{12}$-Alkyl stehen oder zusammen eine der folgenden Alkylenbrücken

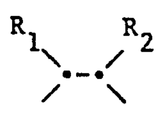 , 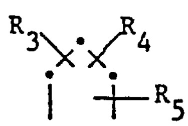 oder 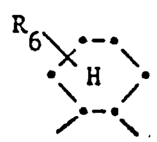 bilden, wobei

$R_1$ und $R_2$ unabhängig voneinander für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_3$-Alkyl substituiertes Phenyl oder für die Gruppe $-CH_2-Z-R_7$ stehen, wobei

Z Sauerstoff oder Schwefel bedeutet und

$R_7$ für Wasserstoff, $C_1$-$C_8$-Alkyl, ein durch $C_1$-$C_2$-Alkoxy substituiertes $C_1$-$C_8$-Alkyl, $C_3$-$C_4$-Alkenyl, 2-Propinyl, 3-Halogen-2-propinyl, Phenyl, ein durch Halogen, $C_1$-$C_3$-Alkyl, $C_1$-$C_3$-Alkoxy, Nitro und/oder $CF_3$ ein- oder mehrfach substituiertes Phenyl, Benzyl oder ein durch Halogen, $C_1$-$C_3$-Alkyl und/oder $C_1$-$C_3$-Alkoxy ein- oder mehrfach substituiertes Benzyl steht;

$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$-$C_3$-Alkyl steht;

unter Einschluss ihrer Säureadditionssalze, dadurch gekennzeichnet, dass man

A) eine Verbindung der Formel II

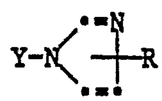

(II) ,

worin Y Wasserstoff oder ein Metallkation darstellt, mit einer Verbindung der Formel III

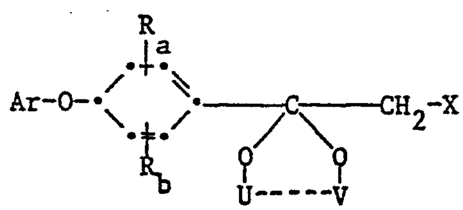

(III),

worin X eine nukleofuge Abgangsgruppe bedeutet, kondensiert oder

B) in einer Verbindung der Formel IV

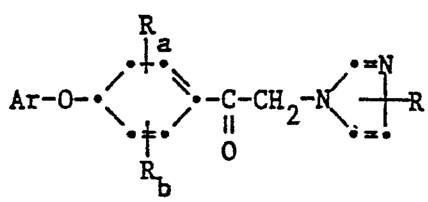

(IV)

die Carbonylgruppe in eine Gruppe der Formel V

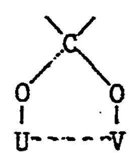

(V)

überführt, oder

C) zur Herstellung von Verbindungen der Formel I, worin U und V gemeinsam eine Gruppe der Formel $-CH_2-CH(CH_2ZR_7')-$ darstellen und $R_7'$ einen von Wasserstoff verschiedenen Rest $R_7$ bedeutet, Verbindungen der Formeln VI und VII

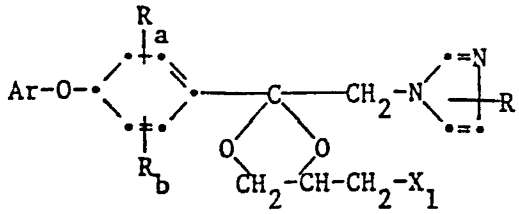 (VI) und $X_2 - R_7$ (VII),

worin einer der Reste $X_1$ und $X_2$ gegebenenfalls in Salzform vorliegendes Hydroxy oder Mercapto, z.B. der Formel -Z-Y, in der Y Wasserstoff oder vorzugsweise ein Metallkation ist, und der andere eine nukleofuge Abgangsgruppe X bedeutet oder sowohl $X_1$ als auch $X_2$ Hydroxygruppen darstellen, miteinander kondensiert oder

D) Verbindungen der Formeln VIII und IX

$Ar-X_3$ (VIII) und 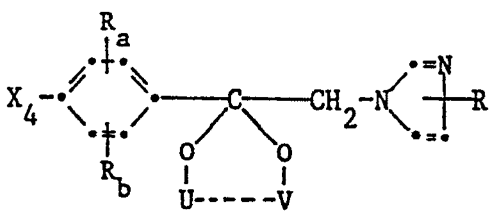 (IX),

worin einer der Reste $X_3$ und $X_4$ eine Gruppe -O-Y, in der Y Wasserstoff oder vorzugsweise ein Metallkation ist, und der andere einen gegen Aryloxy austauschbaren Rest bedeutet, miteinander kondensiert oder

E) eine Verbindung der Formel

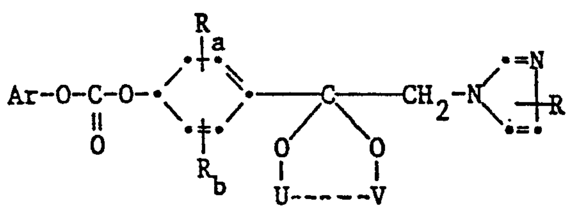 (X)

intramolekular decarboxyliert und, wenn erwünscht, eine verfahrensgemäss erhältliche Verbindung in eine andere Verbindung der Formel I umwandelt und/oder eine verfahrensgemäss erhältliche freie Verbindung in ein Säureadditionssalz, ein verfahrensgemäss erhältliches Säureadditionssalz in die freie Verbindung oder in ein anderes Säureadditionssalz überführt.

2. Verfahren gemäss Anspruch 1, dadurch gekennzeichnet, dass man Verbindungen der Formeln II und III ausgeht, worin Y ein Alkalimetall- oder Erdalkalimetallkation und X eine reaktionsfähige veresterte Hydroxygruppe, z.B. mit einer Halogenwasserstoffsäure, einer Niederalkyl- oder gegebenenfalls substituierten Benzolsulfonsäure oder mit Fluorsulfonsäure verestertes Hydroxy, bedeutet, oder eine Verbindung der Formel IV mit einem Orthocarbonsäure-tri-$C_1$-$C_{12}$-alkylester, dessen $C_1$-$C_{12}$-Alkylgruppen gegebenenfalls durch Halogen oder $C_1$-$C_6$-Alkoxy substituiert sind, oder in Gegenwart einer Säure mit mindestens 2 Mol eines einwertigen Alkohols der Formel U-OH (Va), wobei Verbindungen der Formel I erhalten werden, worin U und V gleiche, gegebenenfalls substituierte $C_1$-$C_{12}$-Alkylgruppen bedeuten, oder mit einem Diol der Formel Vb

HO-U----V-OH (Vb) ,

wobei Verbindungen der Formel I erhalten werden, worin U und V gemeinsam eine der in Anspruch 1 definierten Alkylenbrücken darstellen, umsetzt oder von Verbindungen der Formeln VI und VII ausgeht, worin $X_1$ eine Gruppe -ZH und $X_2$ eine reaktionsfähige veresterte Hydroxygruppe, z.B. mit einer Halogenwasserstoffsäure, einer Niederalkan- oder gegebenenfalls substituierten Benzolsulfonsäure oder mit Fluorsulfonsäure verestertes Hydroxy, bedeutet, oder von Verbindungen der Formel VIII und IX ausgeht, worin einer der Reste $X_3$ und $X_4$ eine Gruppe -OY und der andere eine reaktionsfähige veresterte Hydroxygruppe, z.B. mit einer Halogenwasserstoffsäure, einer Niederalkan- oder gegebenenfalls substituierten Benzolsulfonsäure oder mit Fluorsulfonsäure verestertes Hydroxy, bedeutet, oder von einer auf beliebiger Verfahrensstufe als Zwischenprodukt erhältlichen Verbindung ausgeht und die fehlenden Verfahrensschritte ausführt, oder einen Ausgangsstoff unter den Reaktionsbedingungen gebildet oder in Form eines Derivates davon, gegebenenfalls eines Salzes, verwendet.

3. Verfahren gemäss einem der Ansprüche 1 und 2, dadurch gekennzeichnet, dass man Verbindungen der Formel I oder deren Säureadditionssalze herstellt, worin R, $R_a$ und $R_b$ die in Anspruch 1 gegebenen Bedeutungen haben, Ar eine Gruppe der Formel

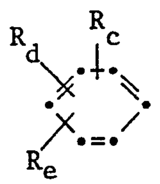

darstellt, in der $R_c$, $R_d$ und $R_e$ unabhängig voneinander Wasserstoff, Halogen, Trifluormethyl, $C_1$-$C_3$-Alkyl oder $C_1$-$C_3$-Alkoxy bedeuten, und U und V unabhängig voneinander $C_1$-$C_6$-Alkyl oder durch Halogen bzw. $C_1$-$C_2$-Alkoxy substituiertes $C_2$-$C_4$-Alkyl oder gemeinsam eine der folgenden Alkylengruppen

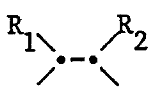

oder

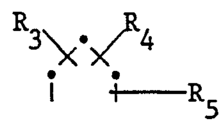

darstellen, worin $R_1$, $R_2$, $R_3$, $R_4$ und $R_5$ unabhängig voneinander Wasserstoff oder $C_1$-$C_4$-Alkyl, wobei die Gesamtzahl der Kohlenstoffatome von $R_3$, $R_4$ und $R_5$ 6 nicht übersteigt oder $R_1$ Wasserstoff und $R_2$ eine Gruppe der Formel $CH_2OR_7$ darstellt, in der $R_7$ $C_1$-$C_4$-Alkyl, durch $C_1$-$C_2$-Alkoxy substituiertes $C_2$-$C_4$-Alkyl, $C_3$-$C_4$-Alkenyl oder Prop-2-inyl bedeutet.

4. Verfahren gemäss einem der Ansprüche 1 und 2, dadurch gekennzeichnet, dass man Verbindungen der Formel I, worin R die unter der Formel I angegebene Bedeutung hat, $R_a$ und $R_b$ unabhängig voneinander Wasserstoff, Methyl, Chlor oder Brom bedeuten, Ar unsubstituiertes oder durch Halogen, Methyl oder Trifluormethyl substituiertes Phenyl bedeutet und U und V unabhängig voneinander $C_1$-$C_3$-Alkyl, gegebenenfalls durch Halogen oder $C_1$-$C_2$-Alkoxy substituiertes $C_2$-$C_3$-Alkyl oder zusammen eine der folgenden Alkylengruppen.

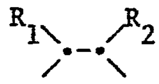

oder

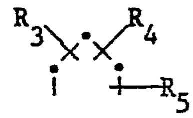

bilden, worin $R_1$, $R_2$, $R_3$, $R_4$ und $R_5$ unabhängig voneinander für Wasserstoff oder $C_1$-$C_4$-Alkyl stehen, wobei die Gesamtzahl der Kohlenstoffatome in $R_3$, $R_4$ und $R_5$ die Zahl 4 nicht übersteigen sollen, oder deren Säureadditionssalze herstellt.

5. Verfahren gemäss einem der Ansprüche 1 und 2, dadurch gekennzeichnet, dass man Verbindungen der Formel I, worin R die unter der Formel I angegebene Bedeutung hat, $R_a$ und $R_b$ Wasserstoff bedeuten, Ar für unsubstituiertes oder durch Halogen oder Methyl substituiertes Phenyl und U sowie V gemeinsam eine Gruppe der Formel

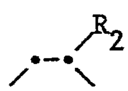

oder

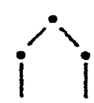

darstellen, in der $R_2$ $C_1$-$C_4$-Alkyl, Hydroxy-$C_1$-$C_3$-alkyl oder $C_1$-$C_2$-Alkoxy-$C_1$-$C_2$-alkyl bedeuten, oder deren Säureadditionssalze herstellt.

6. Verfahren gemäss einem der Ansprüche 1 und 2, dadurch gekennzeichnet, dass man 2-[p-(p-Chlorphenoxy)phenyl]-2-[1-(2-methyl-imidazolyl)methyl]-4-ethyl-1,3-dioxolan oder ein Säureadditionssalz davon herstellt.

7. Verfahren gemäss einem der Ansprüche 1 und 2, dadurch gekennzeichnet, dass man 2-(p-Phenoxyphenyl)-2-[1-(2-methylimidazolyl)-methyl]-4-methyl-1,3-dioxan oder

2-[p-(2,4-Dichlorphenoxy)phenyl]-2-[1-(2-methylimidazolyl)methyl]-4-ethyl-1,3-dioxolan oder ein Säureadditionssalz davon herstellt.

8. Verfahren gemäss einem der Ansprüche 1 und 2, dadurch gekennzeichnet, dass man 2-[4-(p-Chlorphenoxy)-2-methyl-phenyl]-2-[1-(2-methylimidazolyl)methyl]-4-methyl-1,3-dioxolan, 2-[4-(p-chlorphenoxy)-2-methyl-phenyl]-2-(1-(2-methylimidazolyl)methyl]-4-ethyl-1,3-dioxolan, 2-[p-(m-chlorpheonxy)phenyl]-2-[1-(2-methylimidazolyl)-methyl]-4-ethyl-1,3-dioxolan, 2-(2-chlor-4-phenoxyphenyl)-2-[1-(2-methylimidazolyl)methyl]-4-ethyl-dioxolan, 2-[p-chlorphenoxy)-phenyl]-2-[1-(2-methylimidazolyl)methyl]-1,3-dioxan, 2-[p-(4-chlor-3-methyl-phenoxy)phenyl]-2-[1-(2-methylimidazolyl)methyl]-4-methoxy-1,3-dioxolan oder 2-(p-phenoxyphenyl)-2-[1-(2-methylimidazolyl)methyl]-4-propyl-dioxolan oder jeweils ein Säureadditionssalz davon herstellt.

9. Verfahren zur Herstellung pharmazeutischer Präparate, dadurch gekennzeichnet, dass man eine gemäss einem der Ansprüche 1 - 8 erhältliche Verbindung mit üblichen pharmazeutischen Hilfsstoffen vermischt.

10. Verfahren zur Herstellung pharmazeutischer Präparate, dadurch gekennzeichnet, dass man

A) eine Verbindung der Formel II

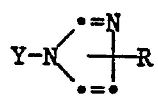

(II) ,

worin Y Wasserstoff oder ein Metallkation darstellt, mit einer Verbindung der Formel III

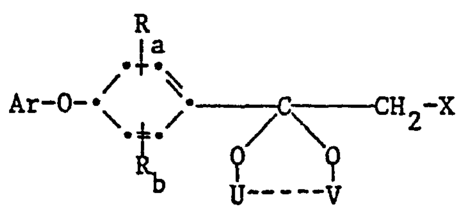

(III),

worin X eine nukleofuge Abgangsgruppe bedeutet, kondensiert oder

B) in einer Verbindung der Formel IV

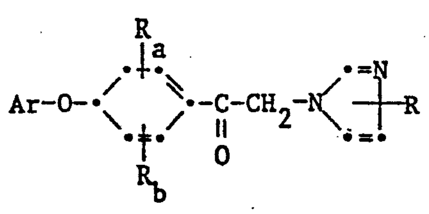

(IV)

die Carbonylgruppe in eine Gruppe der Formel V

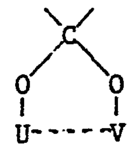
(V)

überführt, oder

C) zur Herstellung von Verbindungen der Formel I, worin U und V gemeinsam eine Gruppe der Formel $-CH_2-CH(CH_2ZR_7')-$ darstellen und $R_7'$ einen von Wasserstoff verschiedenen Rest $R_7$ bedeutet, Verbindungen der Formeln VI und VII

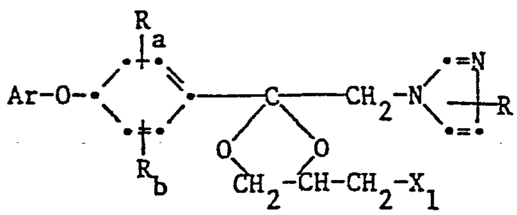
(VI) und $X_2 - R_7$ (VII),

worin einer der Reste $X_1$ und $X_2$ gegebenenfalls in Salzform vorliegendes Hydroxy oder Mercapto, z.B. der Formel -Z-Y, in der Y Wasserstoff oder vorzugsweise ein Metallkation ist, und der andere eine nukleofuge Abgangsgruppe X bedeutet oder sowohl $X_1$ als auch $X_2$ Hydroxygruppen darstellen, miteinander kondensiert oder

D) Verbindungen der Formeln VIII und IX

$Ar-X_3$ (VIII) und 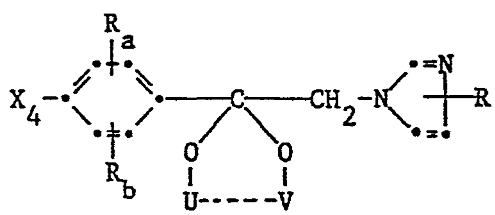
(IX),

worin einer der Reste $X_3$ und $X_4$ eine Gruppe -O-Y, in der Y Wasserstoff oder vorzugsweise ein Metallkation ist, und der andere einen gegen Aryloxy austauschbaren Rest bedeutet, miteinander kondensiert oder

E) eine Verbindung der Formel

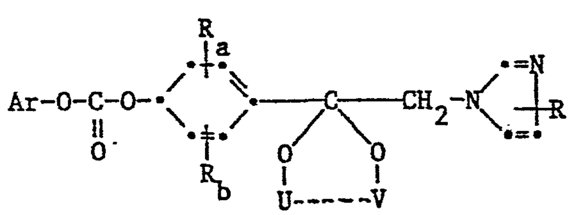
(X)

intramolekular decarboxyliert und, wenn erwünscht, eine verfahrensgemäss erhältliche Verbindung in eine andere Verbindung der Formel I umwandelt und/oder eine verfahrensgemäss erhältliche freie Verbindung in ein Säureadditionssalz, ein verfahrensgemäss erhältliches Säureadditionssalz in die freie Verbindung oder in ein anderes Säureadditionssalz überführt und eines der erhaltenen Arylphenylether-derivate der Formel I

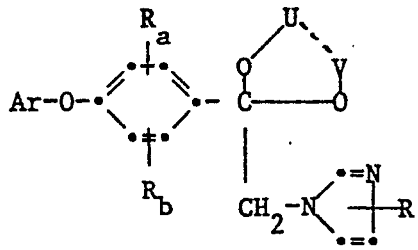 (I) ,

worin

R für $C_1$-$C_6$-Alkyl steht;

$R_a$ und $R_b$ unabhängig voneinander Wasserstoff, Halogen oder $C_1$-$C_3$-Alkyl bedeuten;

Ar unsubstituiertes oder durch Halogen, $C_1$-$C_3$-Alkyl, $C_1$-$C_3$-Alkoxy und/oder Trifluormethyl ein- oder mehrfach substituiertes Phenyl bedeutet;

U und V unabhängig voneinander für gegebenenfalls durch Halogen oder $C_1$-$C_6$-Alkoxy substituiertes $C_1$-$C_{12}$-Alkyl stehen oder zusammen eine der folgenden Alkylenbrücken

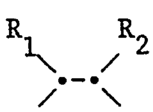 , 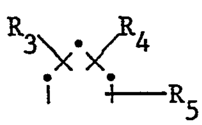 oder 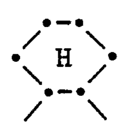 bilden, wobei

$R_1$ und $R_2$ unabhängig voneinander für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_3$-Alkyl substituiertes Phenyl oder für die Gruppe $-CH_2$-Z-$R_7$ stehen, wobei

Z Sauerstoff oder Schwefel bedeutet und

$R_7$ für Wasserstoff, $C_1$-$C_8$-Alkyl, ein durch $C_1$-$C_2$-Alkoxy substituiertes $C_1$-$C_8$-Alkyl, $C_3$-$C_4$-Alkenyl, 2-Propinyl, 3-Halogen-2-propinyl,

Phenyl, ein durch Halogen, $C_1$-$C_3$-Alkyl, $C_1$-$C_3$-Alkoxy, Nitro und/oder $CF_3$ ein oder mehrfach substituiertes Phenyl, Benzyl oder ein durch Halogen, $C_1$-$C_3$-Alkyl und/oder $C_1$-$C_3$-Alkoxy ein- oder mehrfach substituiertes Benzyl steht;

$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$-$C_3$-Alkyl steht;

oder eines seiner Säureadditionssalze mit üblichen pharmazeutischen Hilfsstoffen vermischt.